(19) Europäisches Patentamt
European Patent Office
Office européen des brevets

(11) Veröffentlichungsnummer: **0 293 718 B1**

(12) # EUROPÄISCHE PATENTSCHRIFT

(45) Veröffentlichungstag der Patentschrift: **03.02.93**

(51) Int. Cl.[5]: **C07C 273/08**, C07C 275/30, C07D 319/06, C07C 215/76, C07C 205/22, A01N 47/22, A01N 47/20, A01N 47/30

(21) Anmeldenummer: **88108239.0**

(22) Anmeldetag: **24.05.88**

Die Akte enthält technische Angaben, die nach dem Eingang der Anmeldung eingereicht wurden und die nicht in dieser Patentschrift enthalten sind.

(54) **Substituierte Aminophenyl-carbamate.**

(30) Priorität: **03.06.87 DE 3718522**
**12.02.88 DE 3804288**

(43) Veröffentlichungstag der Anmeldung:
**07.12.88 Patentblatt 88/49**

(45) Bekanntmachung des Hinweises auf die Patenterteilung:
**03.02.93 Patentblatt 93/05**

(84) Benannte Vertragsstaaten:
**BE CH DE ES FR GB IT LI NL**

(56) Entgegenhaltungen:
**EP-A- 0 117 024**
**EP-A- 0 125 901**
**CH-A- 616 654**

**CHEMICAL ABSTRACTS, Band 108, Nr. 14, 4. April 1988, Columbus, Ohio, USA; HARADA,S., "Preparation of m-aminophenol derivatives as materials for pharmaceuticals, agrochemicals, and dyes." Seite 701, Spalte 2/1, Zusammenfassung Nr. 131 277**

(73) Patentinhaber: **BAYER AG**

**W-5090 Leverkusen 1 Bayerwerk(DE)**

(72) Erfinder: **Krüger, Bernd-Wieland, Dr.**
**Sillerstrasse 49**
**W-5600 Wuppertal(DE)**
Erfinder: **Sasse, Klaus, Dr.**
**Pützweg 13**
**W-5060 Bergisch-Gladbach 2(DE)**
Erfinder: **Heitkämper, Peter, Dr.**
**Fliederweg 14**
**W-4047 Dormagen 11(DE)**
Erfinder: **König, Klaus, Dr.**
**Zum Hahnenberg 40**
**W-5068 Odenthal(DE)**
Erfinder: **Brandes, Wilhelm, Dr.**
**Eichendorffstrasse 3**
**W-5653 Leichlingen(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

Erfinder: **Hänssler, Gerd, Dr.**
**Am Arenzberg 58a**
**W-5090 Leverkusen 3(DE)**
Erfinder: **Marhold, Albrecht, Dr.**
**Carl-Duisberg-Strasse 329**
**W-5090 Leverkusen(DE)**

**Beschreibung**

Die vorliegende Erfindung betrifft neue Aminophenyl-carbamate, Verfahren zu ihrer Herstellung und ihre Verwendung zur Bekämpfung von Schädlingen.

Es ist bereits bekannt, daß bestimmte substituierte 3-Aminophenyl-carbamate gute herbizide Eigenschaften besitzen (vgl. US 3 832 389).

Weiterhin sind viele Phenylcarbamate bekannt, die eine fungizide Wirkung besitzen (vgl. EP 116 409; EP 117 024 und EP 125 901).

Weitere substituierte Aminophenyl-carbamate sind in der Humanmedizin beschrieben (vgl. JP 57 007 459).

Es wurden neue Aminophenyl-carbamate der allgemeinen Formel (I)

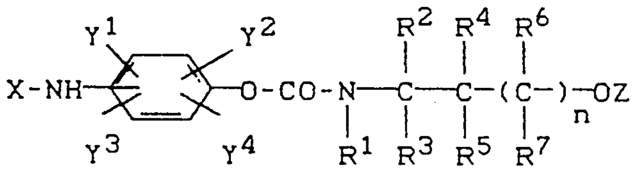

(I)

in welcher

| | |
|---|---|
| X | für -CO-NR$^8$R$^9$; -CO-OR$^9$; -CO-SR$^9$; -CO-R$^{10}$; -SO$_2$R$^9$ oder Wasserstoff steht, worin |
| R$^8$ | für Wasserstoff oder geradkettiges oder verzweigtes Alkyl mit 1 bis 6 Kohlenstoffatomen steht, |
| R$^9$ | für Aryl mit 6 bis 12 kohlenstoffatomen, für ein- bis sechsfach, gleich oder verschieden durch Halogen, Alkyl mit 1 bis 6 Kohlenstoffatomen, Alkoxy mit 1 bis 6 Kohlenstoffatomen, Alkylthio mit 1 bis 6 Kohlenstoffatomen, Halogenalkyl mit 1 bis 6 Kohlenstoffatomen und 1 bis 9 gleichen oder verschiedenen Halogenatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Cyano und Nitro substituiertes Aryl mit 6 bis 12 Kohlenstoffatomen steht; ferner für Aralkyl mit 6 bis 12 Kohlenstoffatomen im Arylteil und mit 1 bis 6 Kohlenstoffatomen im geradkettigen oder verzweigten Alkylteil steht, wobei der Arylrest ein- bis sechsfach, gleich oder verschieden durch die obengenannten Arylsubstituenten substituiert sein kann; ferner für geradkettiges oder verzweigtes Alkyl mit 1 bis 20 Kohlenstoffatomen steht; ferner für ein- bis vierfach, gleich oder verschieden durch Halogen, Alkoxy mit 1 bis 6 Kohlenstoffatomen und Alkylthio mit 1 bis 6 Kohlenstoffatomen substituiertes Alkyl mit 1 bis 20 Kohlenstoffatomen steht, wobei die Alkylteile jeweils geradkettig oder verzweigt sein können; weiterhin für geradkettiges oder verzweigtes Alkenyl mit 2 bis 16 Kohlenstoffatomen oder für ein- bis sechsfach, gleich oder verschieden durch Halogen substituiertes Alkenyl mit 2 bis 6 Kohlenstoffatomen steht; weiterhin-für Cycloalkyl mit 3 bis 10 Kohlenstoffatomen oder für Cycloalkyl-alkyl mit 1 bis 10 Kohlenstoffatomen im geradkettigen oder verzweigten Alkylteil und 3 bis 10 Kohlenstoffatomen im Cycloalkylteil steht, wobei die cyclischen Teile jeweils unabhängig, gleich oder verschieden, ein- bis sechsfach substituiert sein können durch Halogen, geradkettiges oder verzweigtes Alkyl mit 1 bis 6 Kohlenstoffatomen und Halogenalkyl mit 1 bis 6 Kohlenstoffatomen und 1 bis 9 gleichen oder verschiedenen Halogenatomen oder R$^9$ weiterhin für Cycloalkenyl mit 5 bis 10 Kohlenstoffatomen steht, das gleich oder verschieden, ein- bis sechsfach substituiert sein kann durch geradkettiges oder verzweigtes Alkyl mit 1 bis 6 Kohlenstoffatomen; |
| R$^{10}$ | die Bedeutung von R$^9$ hat oder für einen Heterocyclus mit 5 bis 7 Ringgliedern steht, wobei außer Kohlenstoff ein oder mehrere Heteroatome enthalten sind, Der Heterocyclus kann ein- bis sechsfach, gleich oder verschieden durch geradkettiges oder verzweigtes Alkyl mit 1 bis 6 Kohlenstoffatomen substituiert sein; |
| Y$^1$ bis Y$^4$ | gleich oder verschieden sind und für Wasserstoff, Halogen, Nitro, Cyano, geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen, Alkoxy, Halogenalkoxy, Alkylthio, Alkylsulfonyl oder Halo- |

genalkylthio mit jeweils 1 bis 6 Kohlenstoffatomen im geradkettigen oder verzweigten Alkylteil und gegebenenfalls 1 bis 9 gleichen oder verschiedenen Halogenatomen stehen;

$R^1$ bis $R^7$ gleich oder verschieden sind und für Wasserstoff, geradkettiges oder verzweigtes Alkyl mit 1 bis 6 kohlenstoffatomen, Halogenalkyl mit 1 bis 6 Kohlenstoffatomen im geradkettigen oder verzweigten Alkylteil und 1 bis 9 gleichen oder verschiedenen Halogenatomen oder für Alkoxyalkyl mit 1 bis 6 Kohlenstoffatomen je geradkettigem oder verzweigtem Alkylteil stehen;

n für 0 oder 1 steht und

Z für geradkettiges oder verzweigtes Alkyl oder Halogenalkyl mit je 1 bis 6 Kohlenstoffatomen und gegebenenfalls 1 bis 9 gleichen oder verschiedenen Halogenatomen oder für den Rest

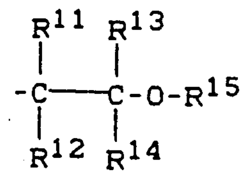

steht, worin

$R^{11}$ bis $R^{14}$ gleich oder verschieden sind und für Wasserstoff, für geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen oder Alkoxyalkyl mit 1 bis 6 Kohlenstoffatomen je geradkettigem oder verzweigtem Alkylteil stehen und

$R^{15}$ für geradkettiges oder verzweigtes Alkyl, Alkoxyalkyl oder Halogenalkyl mit je 1 bis 6 Kohlenstoffatomen je Alkylrest und gegebenenfalls 1 bis 9 gleichen oder verschiedenen Halogenatomen steht,

gefunden.

Die substituierten Aminophenyl-carbamate der Formel (I) enthalten ein oder mehrere Asymmetriezentren und können somit in Form von Diastereomeren oder Diastereomerengemischen vorliegen, die in unterschiedlichen Mengenverhältnissen anfallen. Vorwiegend fallen sie als Racemate an.

Weiterhin wurde gefunden, daß man die neuen substituierten Aminophenyl-carbamate der Formel (I)

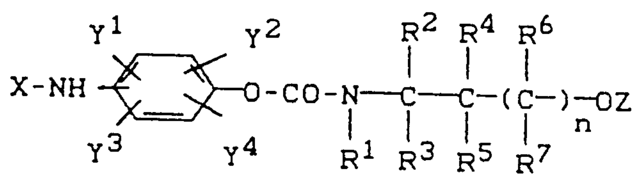

(I)

in welcher

n, X, $Y^1$-$Y^4$, Z und $R^1$-$R^7$ die oben angegebenen Bedeutungen haben erhält, wenn man Aminophenole der Formel (II)

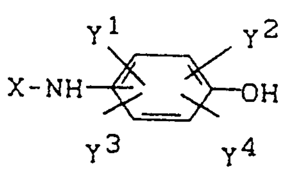

(II)

in welcher

X und $Y^1$ bis $Y^4$ die oben angegebenen Bedeutungen haben,

a) mit Isocyanaten der Formel (III)

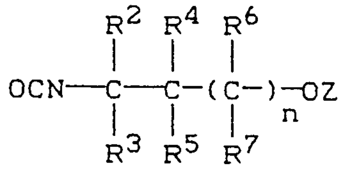

(III)

in welcher

$R^2$ bis $R^7$, n und Z die oben angegebenen Bedeutungen haben, gegebenenfalls in Gegenwart einer Base und gegebenenfalls in Gegenwart von Verdünnungs- oder Lösungsmitteln umsetzt, oder

b) mit Halogencarbonylverbindungen der Formel (IV)

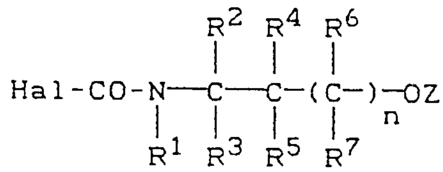

(IV)

in welcher

$R^1$ bis $R^7$, n und Z die oben angegebenen Bedeutungen haben, und

Hal für Halogen, vorzugsweise Chor steht,

gegebenenfalls in Gegenwart eines Säureakzeptors und gegebenenfalls in Gegenwart eines Lösungs- oder Verdünnungsmittels umsetzt.

Schließlich wurde gefunden, daß die substituierten Aminophenyl-carbamate der Formel (I) eine gute Wirkung gegen Schädlinge besitzen, vor allem eine hohe fungizide Wirksamkeit. Die neuen Verbindungen können auch mit anderen bekannten, hochwirksamen Verbindungen in synergistischen Mischungen verwendet werden.

Im Rahmen der vorliegenden Erfindung können die Substituenten im allgemeinen die folgenden Bedeutungen haben:

Halogen kann, überall wo nicht anders angegeben, Fluor, Chlor, Brom und Iod, bevorzugt Fluor, Chlor und Brom, bedeuten.

Gegebenenfalls substituiertes Alkyl steht im allgemeinen für einen geradkettigen oder verzweigten Kohlenwasserstoffrest mit 1 bis 20 Kohlenstoffatomen, bevorzugt mit 1 bis 18 Kohlenstoffatomen. Beispielhaft seien genannt Methyl, Ethyl, n- und iso-Propyl, n-, sec.-. iso- und tert.-Butyl, Hexyl, Heptyl, Dodecyl, Oktadecyl.

Als Substituent eines Alkylrestes seien beispielsweise Halogen, wie oben definiert, genannt, außerdem Alkoxy und Alkylthio.

Alkoxy bzw. Alkylthio bzw. Alkylsulfonyl stehen im allgemeinen für einen über Sauerstoff bzw. Schwefel bzw. $SO_2$ gebundenen, geradkettigen oder verzweigten Kohlenwasserstoffrest mit 1 bis 6, vorzugsweise 1 bis 4 und besonders bevorzugt mit 1 oder 2 Kohlenstoffatomen im Alkoxy- bzw. Alkylthio- bzw. Alkylsulfonylrest allein oder in Zusammensetzungen.

Beispielsweise seien für die substituierten Alkylreste genannt: Fluormethyl, Chlormethyl, Brommethyl, Fluorethyl, Chlorethyl, Bromethyl, Fluorpropyl, Chlorpropyl, Brompropyl, Fluorbutyl, Chlorbutyl, Brombutyl, Fluorisopropyl, Chlorisopropyl, Bromisopropyl, Difluormethyl, Trifluormethyl, Dichlormethyl, Trichlormethyl, Difluorethyl, Dichlorethyl, Trifluorethyl, Tetrafluorethyl, Difluorbutyl, Dichlorbutyl, Trichlorethyl und Trifluorpropyl. Ganz besonders bevorzugt sind Trifluormethyl, 1-Fluor-2-fluormethyl-prop-(2)yl und 1-Chlor-2-methylprop(2)yl.Ferner Methoxymethyl, Methoxyethyl, Methoxy Methoxypropyl, Methoxybutyl, Methoxypentyl, Ethoxymethyl, Ethoxyethyl, Ethoxypropyl, Ethoxybutyl, Ethoxypentyl, Propoxymethyl, Propoxyethyl, Propoxypropyl, Propoxybutyl, Propoxypental, Butoxymethyl, Butoxyethyl, Butoxypropyl, Butoxybutyl und Butoxypentyl. Besonders bevorzugt ist: 2-Methoxyprop-(2)yl.

Ferner Methylthiomethyl, Methylthioethyl, Methylthiopropyl, Methylthiobutyl, Methylthiopentyl, Ethylthiomethyl, Ethylthioethyl, Ethylthiopropyl, Ethylthiobutyl, Ethylthiopentyl, Propylthiomethyl, Propylthioethyl, Propylthiopropyl, Propylthiobutyl, Propylthiopentyl, Butylthiomethyl, Butylthioethyl, Butylthiopropyl, Butylthiobutyl und Butylthiopentyl, Methylsulfonyl, Ethylsulfonyl und Propylsulfonyl.

Alkyl, Alkoxy und Alkoxyalkyl stehen für einen Rest, der je Alkyleinheit 1 bis 6, vorzugsweise 1 bis 4

Kohlenstoffatome hat. Die Beispiele entsprechen den obengenannten.

Halogenalkoxy bzw. Halogenalkylthio steht im allgemeinen für einen über Sauerstoff bzw. Schwefel gebundenen, geradkettigen oder verzweigten Kohlenwasserstoffrest mit 1 bis 6 Kohlenstoffatomen und 1 bis 9 gleichen oder verschiedenen Halogenatomen. Bevorzugt sind Reste mit 1 bis 4 Kohlenstoffatomen und 1 bis 5 gleichen oder verschiedenen Halogenatomen. Ganz besonders bevorzugt sind Reste mit 1 oder 2 Kohlenstoffatomen und 1 bis 3 gleichen oder verschiedenen Halogenatomen. Beispielhaft seien genannt: Trifluormethoxy, Trichlormethoxy, Difluorchlormethoxy, Dichlorfluormethoxy, Difluorethoxy, Trifluorethoxy, Tetrafluorethoxy, Pentafluorethoxy, Trifluormethylthio, Trichlormethylthio, Difluorchlormethylthio, Dichlorfluormethylthio, Difluorethylthio, Trifluormethylthio, Tetrafluorethylthio.

Alkenyl steht im allgemeinen für einen geradkettigen oder verzweigten Kohlenwasserstoffrest mit 2 bis 16 Kohlenstoffatomen und einer oder mehreren, bevorzugt mit einer oder zwei, Doppelbindungen. Bevorzugt ist Niederalkenyl mit 2 bis 10 Kohlenstoffatomen und einer Doppelbindung. Besonders bevorzugt ist ein Alkenylrest mit 2 bis 4 Kohlenstoffatomen und einer Doppelbindung. Beispielsweise seien genannt: Vinyl, Allyl, Propenyl, Isopropenyl, Butenyl, Isobutenyl, Pentenyl, Isopentenyl, Hexenyl, Isohexenyl, Heptenyl, Isoheptenyl, Octenyl und Isooctenyl.

Halogenalkenyl steht im allgemeinen für geradkettiges oder verzweigtes Alkyl mit 1 bis 6 Kohlenstoffatomen und einem bis mehreren, gleichen oder verschiedenen Halogenatomen und mit einer oder mehreren Doppelbindungen Bevorzugt sind Reste mit 1 bis 4 Kohlenstoffatomen, 1 bis 4 gleichen oder verschiedenen Halogenatomen und einer Doppelbindung. Beispielhaft seien genannt: 2,2-Dichlorvinyl, 1,2,2-Trichlorvinyl.

Aryl kann für einen aromatischen Kohlenwasserstoffrest mit 6 bis 12 Kohlenstoffatomen stehen. Beispielsweise seien Phenyl, Naphthyl und Biphenyl genannt. Bevorzugt ist Phenyl.

Aralkyl kann für einen Rest mit 7 bis 18 Kohlenstoffatomen stehen, wobei ein geradkettiger oder verzweigter Alkylrest mit 1 bis 6, bevorzugt 1 bis 4 Kohlenstoffatomen durch einen aromatischen Rest mit 6 bis 12, bevorzugt 6, Kohlenstoffatomen substituiert sein kann. Beispielsweise seien Benzyl, Phenylethyl und Phenylpropyl genannt. Bevorzugt ist Benzyl.

Die Aryl- und Aralkylreste können gegebenenfalls einbis mehrfach, gleich oder verschieden substituiert sein. Als Substituenten seien beispielsweise Halogen, Alkyl mit 1 bis 6 Kohlenstoffatomen, Alkoxy mit 1 bis 6 Kohlenstoffatomen, Alkylthio mit 1 bis 6 Kohlenstoffatomen, Halogenalkyl mit 1 bis 6 Kohlenstoffatomen und 1 bis 9 gleichen oder verschiedenen Halogenatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Cyano und Nitro. Diese Reste haben die bevorzugte und besonders bevorzugte Bedeutung wie bereits oben beschrieben.

Cycloalkyl steht im allgemeinen für einen cyclischen Kohlenwasserstoffrest mit 3 bis 10 Kohlenstoffatomen. Bevorzugt sind der Cyclopropyl-, Cyclopentyl- und Cyclohexylrest. Beispielhaft seien genannt: Cyclopropyl, Cyclobutyl, Cyclopentyl, Cyclohexyl, Cyclodecanyl.

Cycloalkyl-alkyl kann für einen Rest mit 4 bis 20 Kohlenstoffatomen stehen, wobei ein geradkettiger oder verzweigter Alkylrest mit 1 bis 10 Kohlenstoffatomen durch einen Cycloalkylrest mit 3 bis 10 Kohlenstoffatomen substituiert sein kann. Beispielsweise seien Cyclopropylmethyl, Cyclopentylmethyl, Cyclohexylmethyl, 1-Cyclohexylethyl, 2-Cyclohexylethyl, Cycloheptylmethyl und 2-Cycloheptylethyl genannt.

Die Cycloalkyl- und Cycloalkyl-alkylreste können einbis mehrfach, gleich oder verschieden substituiert sein. Als Substituenten seien beispielsweise Halogen, Alkyl mit 1 bis 6 Kohlenstoffatomen und Halogenalkyl mit 1 bis 6 Kohlenstoffatomen und 1 bis 9 gleichen oder verschiedenen Halogenatomen genannt. Die Substituenten haben die bevorzugte und besonders bevorzugte Bedeutung, die bereits weiter oben für die Reste gegeben wurde.

Cycloalkenyl kann für einen cyclischen Kohlenwasserstoffrest mit 5 bis 10 Kohlenstoffatomen und mit einer bis mehreren Doppelbindungen stehen. Bevorzugt sind Reste mit 5 oder 6 Kohlenstoffatomen und einer Doppelbindung. Der Cycloalkenylrest kann ein- bis mehrfach, gleich oder verschieden substituiert sein durch Alkyl mit 1 bis 6 Kohlenstoffatomen. Die bevorzugte und besonders bevorzugte Definition des Alkylrestes entspricht der weiter oben gegebenen.

Heterocyclus kann für einen Rest mit 5 bis 7 Ringgliedern, bevorzugt mit 5 oder 6 Ringgliedern, stehen, wobei außer Kohlenstoff ein oder mehrere Heteroatome, wie Sauerstoff, Schwefel oder Stickstoff, enthalten sind. Bevorzugt sind 5- oder 6-Ringe mit einem oder zwei Sauerstoffatomen. Beispielsweise seien genannt: Oxolanyl, Oxanyl, Dioxolanyl und Dioxanyl. Die Heterocyclen können ein- bis mehrfach, gleich oder verschieden substituiert sein durch Alkyl mit 1 bis 6, vorzugsweise 1 bis 4 und besonders bevorzugt mit 1 oder 2 Kohlenstoffatomen. Beispielsweise seien genannt: 2-Methyl-oxolan-2-yl, 2-Methyl-oxan-2-yl, 5-Methyl-1,3-dioxolan-5-yl, 2-Ethyl-oxolan-2-yl, 2-Ethyl-oxan-2-yl, 5-Ethyl-1,3-dioxolan-5yl.

Die erfindungsgemäßen substituierten Aminophenyl-carbamate sind durch die Formel (I) allgemein

definiert. Bevorzugt sind Verbindungen der Formel (I), worin

X für $-CO-NR^8R^9$; $-CO-OR^9$; $-CO-R^{10}$; $-SO_2R^9$ oder Wasserstoff steht, worin

$R^8$ für Wasserstoff steht,

$R^9$ für gegebenenfalls ein- bis dreifach, gleich oder verschieden im Phenylteil durch Halogen, geradkettig oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Halogenalkyl, Halogenalkoxy oder Halogenalkylthio mit jeweils 1 bis 4 Kohlenstoffatomen im geradkettigen oder verzweigten Alkylteil und 1 bis 5 gleichen oder verschiedenen Halogenatomen, Cyano oder Nitro jeweils substituiertes Phenyl, Benzyl, Phenylethyl oder 1-Phenyl-1-methylethyl steht; für geradkettiges oder verzweigtes Alkyl mit 1 bis 18 Kohlenstoffatomen; für ein- bis dreifach, gleich oder verschieden durch Halogen, Alkoxy oder Alkylthio substituiertes Alkyl mit 1 bis 4 Kohlenstoffatomen im geradkettigen oder verzweigten Alkoxy- oder Alkylthioteil und 1 bis 10 Kohlenstoffatomen im Alkylteil; für geradkettiges oder verzweigtes Alkenyl mit 2 bis 8 Kohlenstoffatomen; für ein- bis dreifach, gleich oder verschieden durch Halogen substituiertes, geradkettiges oder verzweigtes Alkenyl mit 2 bis 4 Kohlenstoffatomen; für gegebenenfalls ein- bis dreifach, gleich oder verschieden durch Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 5 gleichen oder verschiedenen Halogenatomen jeweils substituiertes Cycloalkyl mit 3 bis 6 Kohlenstoffatomen oder Cycloalkyl-alkyl mit 3 bis 6 Kohlenstoffatomen im Cycloalkylteil und 1 bis 6 Kohlenstoffatomen im geradkettigen oder verzweigten Alkylteil steht;

$R^{10}$ die Bedeutung von $R^9$ hat oder für einen gegebenenfalls ein- bis dreifach durch geradkettiges oder verzweigtes Alkyl mit 1 bis 4 Kohlenstoffatomen substituierten 5- oder 6-gliedrigen Heterocyclus mit einem oder zwei Sauerstoffatomen steht,

$Y^1$ bis $Y^4$ gleich oder verschieden sind und für Wasserstoff, Halogen, geradkettiges oder verzweigtes Alkyl mit 1 bis 4 Kohlenstoffatomen, geradkettiges oder verzweigtes Alkoxy bzw. Alkylsulfonyl mit 1 je bis 4 Kohlenstoffatomen oder Halogenalkyl mit 1 bis 4 Kohlenstoffatomen im geradkettigen oder verzweigten Alkylteil und 1 bis 5 gleichen oder verschiedenen Halogenatomen stehen;

$R^1$ bis $R^7$ gleich oder verschieden sind und für Wasserstoff oder geradkettiges oder verzweigtes Alkyl mit 1 bis 6 Kohlenstoffatomen stehen,

n für 0 steht und

Z für geradkettiges oder verzweigtes Alkyl oder Halogenalkyl mit je 1 bis 4 Kohlenstoffatomen und gegebenenfalls 1 bis 5 gleichen oder verschiedenen Halogenatomen oder für den Rest

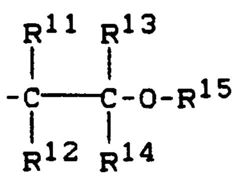

steht, worin

$R^{11}$ bis $R^{14}$ gleich oder verschieden sind und für Wasserstoff, geradkettiges oder verzweigtes Alkyl oder Halogenalkyl mit jeweils 1 bis 4 Kohlenstofatomen und gegebenenfalls 1 bis 5 gleichen oder verschiedenen Halogenatomen steht und

$R^{15}$ für geradkettiges oder verzweigtes Alkyl, Alkoxyalkyl oder Halogenalkyl mit jeweils 1 bis 4 Kohlenstoffatomen je Alkylrest und gegebenenfalls 1 bis 5 gleichen oder verschiedenen Halogenatomen steht.

Ganz besonders bevorzugt sind Verbindungen der Formel (I), worin

X für $-CO-NHR^9$; $-CO-OR^9$; $-CO-R^{10}$ oder $-SO_2R^9$ steht, worin

$R^9$ für gegebenenfalls ein- bis dreifach, gleich oder verschieden durch Methyl, Ethyl, Methoxy, Ethoxy, Trichlormethyl, Trifluormethyl, Trichlormethoxy, Trifluormethoxy, Fluor und Chlor substituiertes Phenyl steht; für Benzyl; für geradkettiges oder verzweigtes Alkyl mit 1 bis 5 Kohlenstoffatomen; für Halogenalkyl mit 1 bis 4 Kohlenstoffatomen und 1 oder

2 Chlor- oder Fluoratomen; für Halogenalkenyl mit 2 oder 3 Kohlenstoffatomen und 1 bis 4 Chloratomen; für gegebenenfalls jeweils ein- bis dreifach, gleich oder verschieden durch geradkettiges oder verzweigtes Alkyl mit 1 bis 4 Kohlenstoffatomen substituiertes Cyclopropyl oder Cyclohexyl steht,

$R^{10}$ für geradkettiges oder verzweigtes Alkyl mit 1 bis 18 Kohlenstoffatomen, für gegebenenfalls durch Methyl, Ethyl, n-Propyl, iso-Propyl, Chlor oder Trifluormethyl ein- bis dreifach, gleich oder verschieden substituiertes Phenyl, Cyclopropyl oder Cyclohexyl; gegebenenfalls ein- oder zweifach durch Chlor substituiertes Phenylalkyl mit 1 bis 3 Kohlenstoffatomen im geradkettigen oder verzweigten Alkylteil; für geradkettiges oder verzweigtes Halogenalkyl mit 1 bis 4 Kohlenstoffatomen und 1 oder 2 Chlor- oder Fluoratome; für geradkettiges oder verzweigtes Halogenalkenyl mit 2 bis 4 Kohlenstoffatomen und 1 bis 4 gleichen oder verschiedenen Halogenatomen, wie Chlor und Fluor, oder für gegebenenfalls einfach durch Methyl substituiertes Oxanyl, Oxolanyl, Dioxanyl oder Dioxolanyl steht;

$Y^1$ bis $Y^4$ gleich oder verschieden sind und für Wasserstoff, Chlor, Fluor, Brom, Methyl t-Butyl, Trifluormethyl oder Methylsulfonyl stehen,

$R^1$ bis $R^7$ gleich oder verschieden sind und für Wasserstoff oder Methyl stehen,

n für 0 steht und

Z für geradkettiges oder verzweigtes Alkyl mit 1 bis 4 Kohlenstoffatomen oder für den Rest

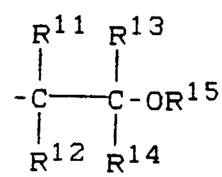

steht, worin

$R^{11}$ bis $R^{14}$ für Wasserstoff und

$R^{15}$ für Methyl, Ethyl, Propyl, Butyl, Methoxyethyl oder Ethoxyethyl steht.

Die Amingruppe kann in 2-, 3- oder 4-Stellung, besonders in 3- oder 4-Stellung und ganz besonders in 4-Stellung zum Carbamatrest stehen.

Verwendet man beispielsweise 2,6-Dichlor-4-(tert.-butylcarbonylamino)-phenol und 2-Methoxyethylisocyanat als Ausgangsstoffe, so kann der Reaktionsablauf der Verfahrensvariante a) durch das folgende Formelschema wiedergegeben werden:

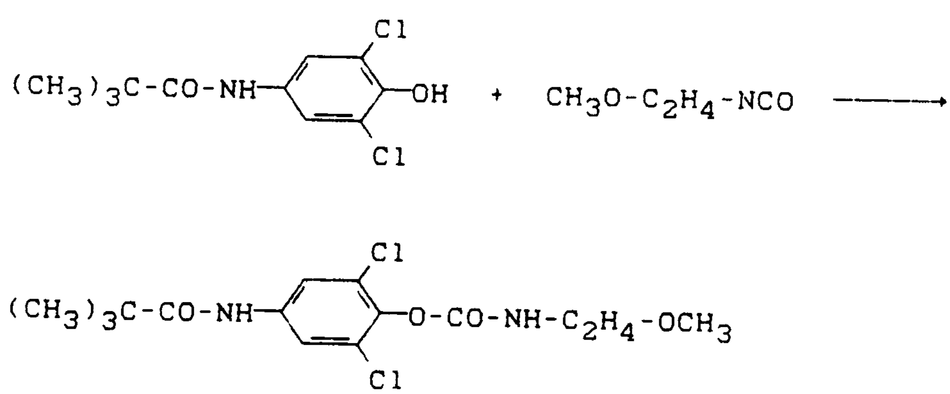

Verwendet man beispielsweise 2,6-Dichlor-4-(methylcarbonylamino)-phenol und N-Methyl-N-(3,6-dioxaheptyl)-carbamidsäurechlorid als Ausgangsstoffe, so kann der Reaktionsablauf der Verfahrensvariante b) durch das folgende Formelschema wiedergegeben werden;

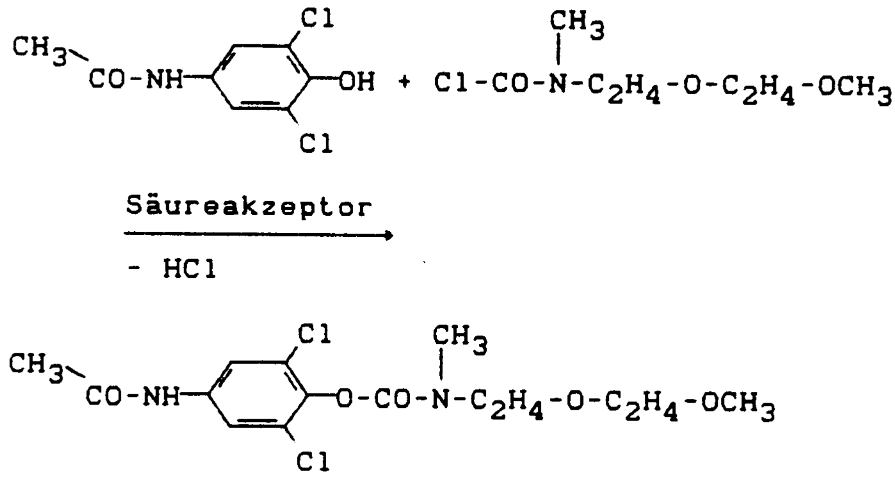


Die zur Durchführung der erfindungsgemäßen Verfahrensvarianten a) und b) als Ausgangsstoffe benötigten Aminophenole sind durch die Formel (II) allgemein definiert. In dieser Formel (II) haben die Reste X und $Y^1$ bis $Y^4$ die bereits im Zusammenhang mit der Beschreibung der erfindungsgemäßen Verbindungen der Formel (I) gegebenen Bedeutungen. Die Verbindungen sind größtenteils bekannt und nach Analogieverfahren herstellbar [vgl. "Methoden der organischen Chemie" Houben-Weyl, Bd. VI/1C Phenole, Teil 1, Georg Thieme Verlag Stuttgart (1976)].

So ist z.B. das 4-Amino-2-trifluormethyl-phenyl bekannt aus J. Org. Chem. 27, 4660 (1962); die 4-Amino-2-chlor- bzw. -2-brom-6-trifluormethylphenole bekannt aus Jp. Kokai Tokkyo Koho Jp 61/126055 und z.B. 4-Amino-2,3,5,6-tetrafluorphenol aus Zh. Org. Khim. 10(9), 1923-1927 (1974). Die z.B. noch neuen Verbindungen der Formel (IIA) bzw. (IIB)

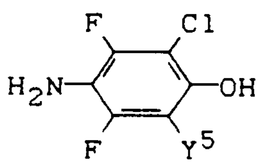 (IIA) bzw. 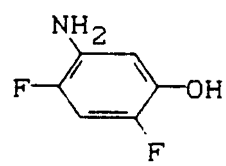 (IIB)

in welcher

$Y^5$ für Fluor oder Chlor steht, können z.B. hergestellt werden aus entsprechenden Hydroxybenzoesäuren der Formel (VA) bzw. (VB)

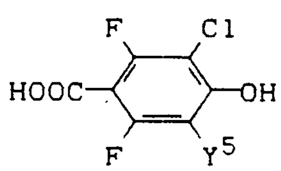 (VA) bzw. 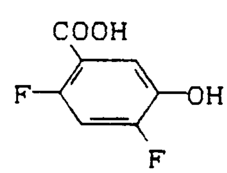 (VB)

durch Decarboxylierung und die entstehenden Phenole der Formeln (VIA) bzw. (VIB)

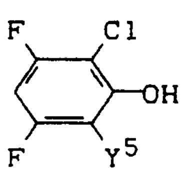 (VIA) bzw. 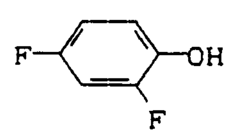 (VIB)

werden nitriert zu den Nitroverbindungen der Formel (VIIA) bzw. VIIB)

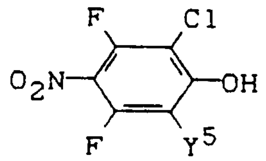 (VIIA) bzw. 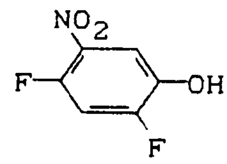 (VIIB)

die dann hydriert werden z.B. mit Wasserstoff und Raney-Nickel zu den entsprechenden Aminen der Formeln (IIA) und (IIB).

Auch die Verbindungen der Formeln (VIIA) und (VIIB) sind erfindungsgemäße Verbindungen.

Die zur Durchführung der erfindungsgemäßen Verfahrensvariante a) außerdem benötigten Isocyanate sind durch die Formel (III) allgemein definiert. In dieser Formel (III) haben die Reste $R^2$ bis $R^7$, n und Z die bereits im Zusammenhang mit der Beschreibung der erfindungsgemäßen Verbindungen der Formel (I) gegebenen Bedeutungen. Die Verbindungen sind bekannt und nach Analogieverfahren herstellbar [vgl. "Methoden der organischen Chemie" Houben-Weyl, Bd. E4, Kohlensäure-Derivate, Georg Thieme Verlag Stuttgart, New York, S. 738 ff (1983)].

Die weiterhin zur Durchführung der erfindungsgemäßen Verfahrensvariante b) benötigten Halogencarbonylverbindungen sind durch die Formel (IV) allgemein definiert. In dieser Formel (IV) haben die Reste Hal, $R^1$ bis $R^7$, n und Z die bereits im Zusammenhang mit der Beschreibung der erfindungsgemäßen Verbindungen der Formel (I) gegebenen Bedeutungen.

Die Verbindungen der Formel (IV) sind größtenteils bekannt oder können nach Analogieverfahren hergestellt werden [vgl. "Methoden der organischen Chemie" Houben-Weyl, Bd. E4, Kohlensäure-Derivate, Georg Thieme Verlag Stuttgart, New York, S. 36 ff (1983)].

Als Verdünnungsmittel zur Durchführung der erfindungsgemäßen Verfahrensvarianten a) und b) kommen praktisch alle inerten organischen Verdünnungsmittel infrage. Hierzu gehören insbesondere aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethyl- und Dibutylether, Glykoldimethylether und Diglykoldimethylether, Tetrahydrofuran und Dioxan, Ketone, wie Aceton, Methylethyl-, Methylisopropyl- und Methylisobutylketon, Ester, wie Essigsäuremethylester und -ethylester, Nitrile, wie z.B. Acetonitril und Propionitril, Amide, wie z.B. Dimethylformamid, Dimethylacetamid und N-Methylpyrrolidon, sowie Dimethylsulfoxid, Tetramethylensulfon und Hexamethylphosphorsäuretriamid.

Die erfindungsgemäßen Verfahrensvarianten a) und b) werden im allgemeinen bei Temperaturen zwischen -50 °C und 120 °C durchgeführt. Bevorzugt wird der Bereich zwischen 0 °C und 110 °C. Die Umsetzungen werden im allgemeinen bei Normaldruck durchgeführt.

Die Verfahrensvariante b) wird gegebenenfalls in Gegenwart von Säureakzeptoren durchgeführt. Als Säureakzeptoren können alle üblichen Säurebindemittel Verwendung finden. Besonders bewährt haben sich Alkalicarbonate und -alkoholate, wie Nartrium- und Kaliumcarbonat, Natrium-und Kaliummethylat bzw. -ethylat, ferner aliphatische, aromatische oder heterocyclische Amine, beispielsweise Triethylamin, Trimethylamin, Dimethylanilin, 1,8-Diazabicyclo(5,4,0)-undec-7-en, Dimethylbenzylamin und Pyridin.

Die Verfahrensvariante a) kann gegebenenfalls in Gegenwart von Basen durchgeführt werden. Als Basen können die bereits bei den Säureakzeptoren aufgeführten Basen Verwendung finden.

Zur Durchführung der erfindungsgemäßen Verfahrensvariante a) setzt man vorzugsweise auf ein Mol Phenol der allgemeinen Formel (II) 1 bis 2 Mol, insbesondere 1 bis 1,4 Mol, der Verbindungen der allgemeinen Formel (III) ein.

Zur Durchführung der erfindungsgemäßen Verfahrensvariante b) setzt man vorzugsweise auf 1 Mol Phenol der allgemeinen Formel (II) 1 bis 2 Mol, insbesondere 1 bis 1,4 Mol, der Verbindungen der Formel (IV) ein.

Die Aufarbeitung erfolgt nach üblichen Methoden, beispielsweise durch Extraktion der Produkte mit Toluol oder Methylenchlorid aus der mit Wasser verdünnten Reaktionsmischung, Waschen der organischen Phase mit Wasser, Trocknen und Destillieren oder sogenanntes "Andestillieren", d.h. längeres Erhitzen unter vermindertem Druck auf mäßig erhöhte Temperaturen, um sie von den letzten flüchtigen Bestandteilen zu befreien, oder durch chromatographische Reinigung über Kieselgel oder z.B. durch Kristallisation. Zur Charakterisierung der Verbindungen dienen Brechungsindex, Schmelzpunkt, $R_f$-Wert oder Siedepunkt.

Die erfindungsgemäßen Wirkstoffe weisen eine starke Wirkung gegen Schädlinge auf und können zur

Bekämpfung von unerwünschten Schadorganismen praktisch eingesetzt werden. Die Wirkstoffe sind für den Gebrauch insbesondere als Fungizide geeignet.

Fungizide Mittel im Pflanzenschutz werden z.B. eingesetzt zur Bekämpfung von Plasmodiophoromycetes, Oomycetes, Chytridiomycetes, Zygomycetes, Ascomycetes, Basidiomycetes, Deuteromycetes.

Bakterizide Mittel werden im Pflanzenschutz z.B. zur Bekämpfung von Pseudomonadaceae, Rhizobiaceae, Enterobacteriaceae, Corynebacteriaceae und Streptomycetaceae eingesetzt.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans;
Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Chochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie

Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier- und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglykol-ether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen, wie Fungizide, Insektizide, Akarizide und Herbizide, sowie in Mischungen mit Düngemitteln und Wachstumsregulatoren.

Die Wirkstoffe können als solche, in Form ihrer Formulierungen oder den daraus bereiteten Anwendungsformen, wie gebrauchsfertige Lösungen, emulgierbare Konzentrate, Emulsionen, Schäume, Suspensionen, Spritzpulver, Pasten, lösliche Pulver, Stäubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Bei der Saatgutbehandlung werden im allgemeinen Wirkstoffmengen von 0,001 bis 50 g je Kilogramm Saatgut, vorzugsweise 0,01 bis 10 g, benötigt.

Bei Behandlung des Bodens sind Wirkstoffkonzentrationen von 0,00001 bis 0,1 Gew.-%, vorzugsweise von 0,0001 bis 0,2 %, am Wirkungsort erforderlich.

## Herstellungsbeispiele

### Beispiel 1

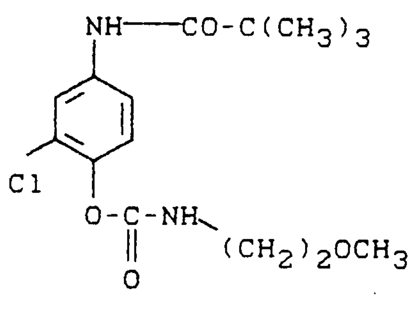

10 g (0,038 Mol) 2-Chlor-4-(tert.-butyl-carbonylamino)-phenol werden in 20 ml Toluol gelöst und mit 4,2 g (0,042 Mol) 2-Methoxyethylisocyanat versetzt. Anschließend fügt man 30 mg (0,2 mMol) 1,8-Diazabicyclo-(5,4,0)-undec-7-en zur gut gerührten Reaktionsmischung. Nach Abklingen der exothermen Reaktion (bis 45 °C) wird noch 2 Stunden bei 20 °C gerührt, auf 0 °C abgekühlt und der ausgefallende Feststoff abgesaugt. Anschließend wird noch einmal in Toluol:Aceton = 7:3 aufgenommen und mit gleichem

Laufmittel über Kieselgel filtriert. Man erhält nach Abdampfen des Lösungsmittels 11,2 g (90 % der Theorie) 2-Methoxyethylcarbamidsäure-[2-chlor-4-(tert.-butylcarbonylamino)]-phenylester mit einem Schmelzpunkt 118 °C.

Analog werden hergestellt:

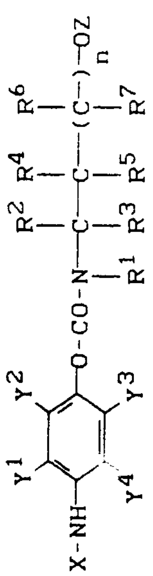

| Beispiel Nr. | X | $Y^1$ | $Y^2$ | $Y^3$ | $Y^4$ | $R^1$ | 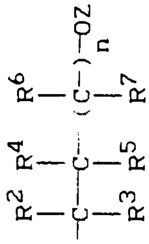 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 2 | $-COC_4H_9$-tert. | H | Cl | Cl | H | H | $-CH_2CH_2OCH_2CH_2OCH_3$ | 100 |
| 3 | $-COC_4H_9$-tert. | H | Cl | H | H | H | $-CH_2CH_2OCH_2CH_2OCH_3$ | 67-73 |
| 4 | $-COC_4H_9$-tert. | Cl | H | H | H | H | $-CH_2CH_2OCH_2CH_2OCH_3$ | 0,45* |
| 5 | $-COOC_2H_5$ | H | Cl | H | H | H | $-CH_2CH_2OCH_2CH_2OCH_3$ | 1,529 |
| 6 | $-COOC_4H_9$-sek. | H | H | H | H | H | $-CH_2CH_2OCH_2CH_2OCH_3$ | 54-55 |
| 7 | $-COOC_3H_7$-i | H | $CH_3$ | H | H | H | $-CH_2CH_2OCH_2CH_2OCH_3$ | 74-76 |
| 8 | $-COOC_2H_5$ | H | $CH_3$ | H | H | H | $-CH_2CH_2OCH_2CH_2OCH_3$ | 57-58 |
| 9 | $-COOC_2H_5$ | H | Cl | Cl | H | H | $-CH_2CH_2OCH_2CH_2OCH_3$ | 1,529 |
| 10 | $-COOC_3H_7$-i | H | Cl | Cl | H | H | $-CH_2CH_2OCH_2CH_2OCH_3$ | 1,527 |
| 11 | $-COOCH_2-$ 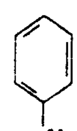 | H | Cl | Cl | H | H | $-CH_2CH_2OCH_2CH_2OCH_3$ | 76-80 |

| Beispiel Nr. | X | $Y^1$ | $Y^2$ | $Y^3$ | $Y^4$ | $R^1$ | 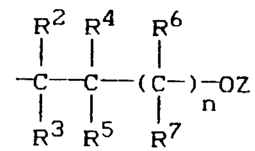 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 12 | H | H | Cl | Cl | H | H | $-CH_2CH_2OCH_2CH_2OCH_3$ | 0,36* |
| 13 | $-CO-NHCH_2-C(CH_3)_3$ | H | Cl | H | H | H | $-CH_2CH_2OCH_2CH_2OCH_3$ | 129-131 |
| 14 | -CO-NH- 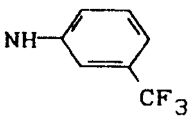 | H | Cl | Cl | H | H | $-CH_2CH_2OCH_2CH_2OCH_3$ | 136-139 |
| 15 | $-CO-NHCH_3$ | H | Cl | Cl | H | H | $-CH_2CH_2OCH_2CH_2OCH_3$ | 190-191 |
| 16 | $-SO_2-$ 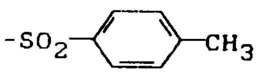 | H | Cl | Cl | H | H | $-CH_2CH_2OCH_2CH_2OCH_3$ | 176 |
| 17 | -CO- 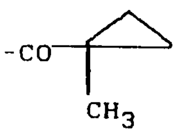 | H | Cl | Cl | H | H | $-CH_2CH_2OCH_2CH_2OCH_3$ | 0,44* |
| 18 | $-CO-CH_3$ | H | Cl | H | H | H | $-CH_2CH_2OCH_2CH_2OCH_3$ | 1,532 |
| 19 | $-CO-CH_3$ | H | Cl | H | H | H | $-CH_2CH_2OCH_2CH_2OCH_3$ | 128 |

| Beispiel Nr. | X | $Y^1$ | $Y^2$ | $Y^3$ | $Y^4$ | $R^1$ | 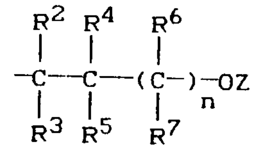 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 20 | 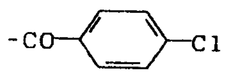 | H | Cl | Cl | H | H | $-CH_2CH_2OCH_2CH_2OCH_3$ | 0,47* |
| 21 | $-CO-C_4H_9$-tert. | H | Br | $CF_3$ | H | H | $-CH_2CH_2OCH_2CH_2OCH_3$ | 0,53* |
| 22 | $-CO-C_4H_9$-tert. | H | $CF_3$ | H | H | H | $-CH_2CH_2OCH_2CH_2OCH_3$ | 0,39* |
| 23 | $-CO-C_4H_9$-tert. | Cl | Cl | H | H | H | $-CH_2CH_2OCH_2CH_2OCH_3$ | 63-65 |
| 24 | $-CO(CH_2)_{16}CH_3$ | H | Cl | Cl | H | H | $-CH_2CH_2OCH_2CH_2OCH_3$ | 47 |
| 25 | 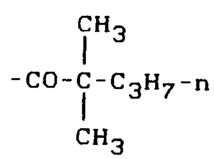 | H | Cl | Cl | H | H | $-CH_2CH_2OCH_2CH_2OCH_3$ | 62-64 |
| 26 | 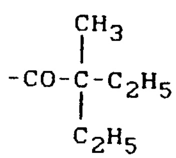 | H | Cl | Cl | H | H | $-CH_2CH_2OCH_2CH_2OCH_3$ | 66-67 |

| Beispiel Nr. | X | $Y^1$ | $Y^2$ | $Y^3$ | $Y^4$ | $R^1$ | 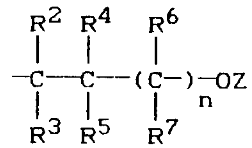 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 27 | 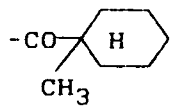 | H | Cl | Cl | H | H | $-CH_2CH_2OCH_2CH_2OCH_3$ | 87-89 |
| 28 | 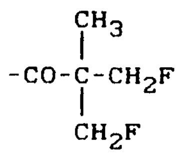 | H | Cl | Cl | H | H | $-CH_2CH_2OCH_2CH_2OCH_3$ | 80-82 |
| 29 | 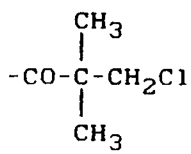 | H | Cl | Cl | H | H | $-CH_2CH_2OCH_2CH_2OCH_3$ | 72-75 |
| 30 | 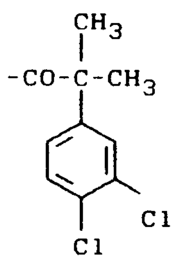 | H | Cl | Cl | H | H | $-CH_2CH_2OCH_2CH_2OCH_3$ | 0,56* |

| Bei-spiel Nr. | X | $Y^1$ | $Y^2$ | $Y^3$ | $Y^4$ | $R^1$ | 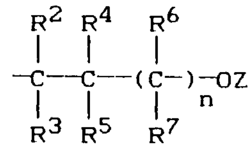 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 31 | 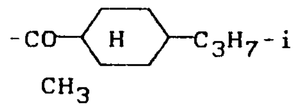 | H | Cl | Cl | H | H | $-CH_2CH_2OCH_2CH_2OCH_3$ | 0,57* |
| 32 | 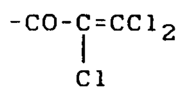 | H | Cl | Cl | H | H | $-CH_2CH_2OCH_2CH_2OCH_3$ | 1,5160 |
| 33 | $-CO-OC_3H_7$-i | Cl | Cl | H | H | H | $-CH_2CH_2OCH_2CH_2OCH_3$ | 98 |
| 34 | $-CO-OC_2H_5$ | Cl | Cl | H | H | H | $-CH_2CH_2OCH_2CH_2OCH_3$ | 95 |
| 35 | $-CO-C_4H_9$-tert. | Cl | Cl | H | H | $CH_3$ | $-CH_2CH_2OCH_2CH_2OCH_3$ | 1,5265 |
| 36 | 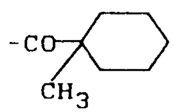 | H | Cl | Cl | H | H | $-CH_2CH_2CH_2OCH_3$ | 136 |
| 37 | 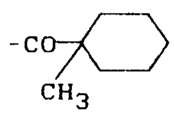 | H | Cl | Cl | H | H | $-CH_2CH_2CH_2OC_4H_9$-n | 105 |

| Beispiel Nr. | X | $Y^1$ | $Y^2$ | $Y^3$ | $Y^4$ | $R^1$ | 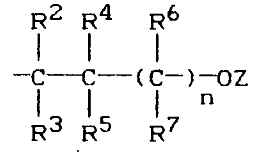 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 38 | -CO 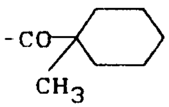 | H | Cl | Cl | H | H | $-CH_2CH_2OCH_2CH_2OC_4H_9$-n | 84 |
| 39 | $-CO-OC_4H_9$-i | Cl | H | H | H | H | $-CH_2CH_2OCH_2CH_2OCH_3$ | 1,5186 |
| 40 | -CO 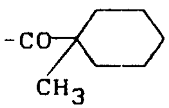 | H | Cl | Cl | H | H | $-CH_2CH_2CH_2OC_2H_5$ | 138-141 |
| 41 | -CO 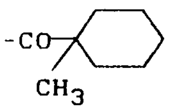 | H | Cl | Cl | H | H | $-CH_2CH_2OCH_3$ | 130 |
| 42 | $-CO-OC_2H_5$ | Cl | H | H | H | H | $-CH_2CH_2OCH_2CH_2OCH_3$ | 1,5328 |
| 43 | -CO 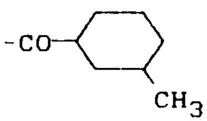 | H | Cl | Cl | H | H | $-CH_2CH_2OCH_3$ | 79 |

| Beispiel Nr. | X | $Y^1$ | $Y^2$ | $Y^3$ | $Y^4$ | $R^1$ | 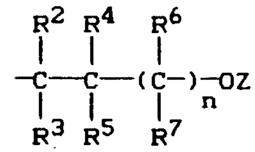 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 44 | 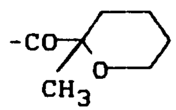 | H | Cl | Cl | H | H | $-CH_2CH_2OCH_3$ | 129-132 |
| 45 | 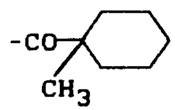 | H | $CF_3$ | Cl | H | H | $-CH_2CH_2OCH_3$ | 114 |
| 46 | $-CO-C_4H_9$-tert. | Cl | Cl | H | H | H | $-CH_2CH_2OCH_3$ | 68-69 |
| 47 | 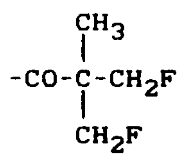 | H | H | Cl | H | H | $-CH_2CH_2OCH_3$ | 122 |
| 48 | 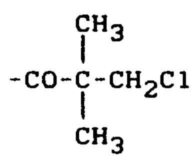 | H | Cl | Cl | H | H | $-CH_2CH_2OCH_3$ | 135 |

| Beispiel Nr. | X | $Y^1$ | $Y^2$ | $Y^3$ | $Y^4$ | $R^1$ | 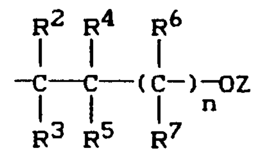 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 49 | 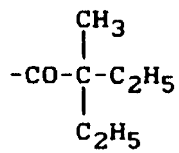 | H | Cl | Cl | H | H | $-CH_2CH_2OCH_3$ | 106-109 |
| 50 | 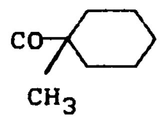 | F | Cl | Cl | F | H | $-CH_2CH_2OCH_3$ | |
| 51 | 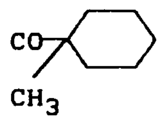 | H | Cl | H | H | H | $-CH_2CH_2OCH_3$ | 100 |
| 52 | 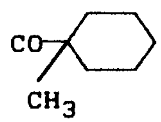 | H | Cl | H | H | H | $CH(CH_3)CH_2OCH_3$ | 88 |

| Beispiel Nr. | X | $Y^1$ | $Y^2$ | $Y^3$ | $Y^4$ | $R^1$ | 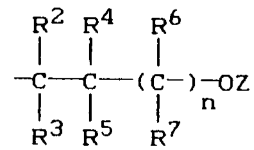 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 53 | 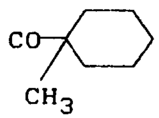 | Cl | Cl | H | H | H | $-CH_2CH_2OCH_3$ | 1,5430 |
| 54 | 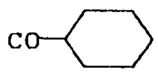 | H | Cl | Cl | H | H | $-CH_2CH_2OCH_3$ | 156 |
| 55 | 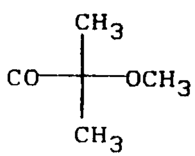 | H | Cl | Cl | H | H | $-CH_2CH_2OCH_3$ | 115 |
| 56 | 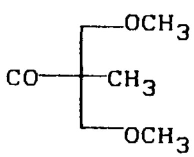 | H | Cl | Cl | H | H | $-CH_2CH_2OCH_3$ | 102 |

| Beispiel Nr. | X | $Y^1$ | $Y^2$ | $Y^3$ | $Y^4$ | $R^1$ | 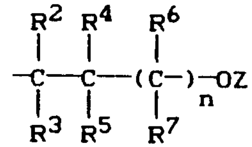 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 57 | 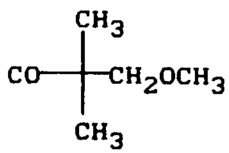 | H | Cl | Cl | H | H | $-CH_2CH_2OCH_3$ | 80 |
| 58 | 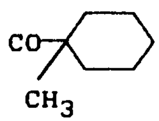 | F | F | Cl | F | H | $-CH_2CH_2OCH_3$ | 148 |
| 59 | 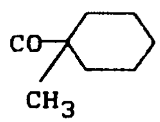 | H | Cl | Cl | H | H | $CH(CH_3)CH_2OC_3H_7$-i | 96 |
| 60 | 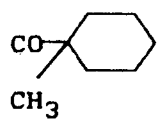 | H | Cl | Cl | H | H | $CH(CH_3)CH_2OC_4H_9$-n | 76 |

| Beispiel Nr. | X | $Y^1$ | $Y^2$ | $Y^3$ | $Y^4$ | $R^1$ | 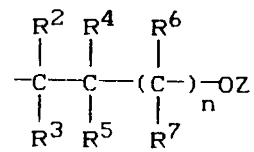 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 61 | 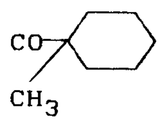 | H | Cl | Cl | H | H | $CH(C_2H_5)CH_2OCH_3$ | 108 |
| 62 | 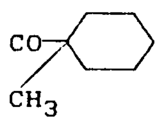 | H | Cl | Cl | H | H | $CH(CH_3)CH_2OCH(CH_3)C_2H_5$ | 108 |
| 63 | 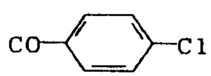 | H | Cl | Cl | H | H | $CH_2CH_2OCH_3$ | 85 |
| 64 | 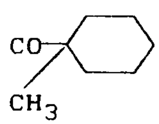 | H | Cl | Cl | H | H | $CH(CH_3)CH_2OC_3H_7$-n | 110 |

| Beispiel Nr. | X | $Y^1$ | $Y^2$ | $Y^3$ | $Y^4$ | $R^1$ | 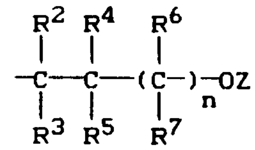 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 65 | 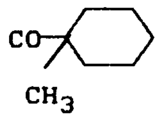 | H | Cl | Cl | H | H | $CH(CH_3)CH_2OC_2H_5$ | 107 |
| 66 | 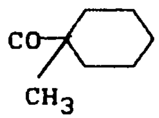 | H | Cl | Cl | H | H | $CH(CH_3)CH_2OCH_3$ | 83 |
| 67 | 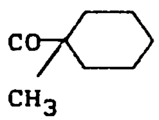 | H | $CH_3$ | H | H | H | $CH_2CH_2OCH_3$ | 115 |
| 68 | 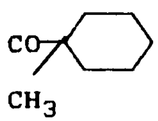 | H | Cl | Cl | H | H | $CH_2CH_2OCH_2CH_2OCH_2CH_2OCH_3$ | 61 |

| Beispiel Nr. | X | $Y^1$ | $Y^2$ | $Y^3$ | $Y^4$ | $R^1$ | 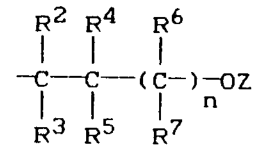 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 69 | 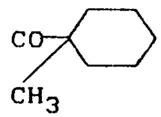 | H | Cl | H | H | H | $CH(CH_3)CH_2OCH_3$ | 88 |
| 70 | 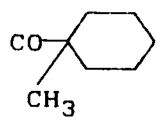 | H | Cl | Cl | H | H | $CH_2CH_2OCH_3$ | 103 |
| 71 | 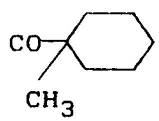 | H | Cl | Cl | H | H | $CH(CH_3)CH_2OCH_2C(CH_3)_3$ | 114 |
| 72 | 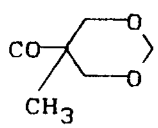 | H | Cl | Cl | H | H | $CH_2CH_2OCH_3$ | 102 |

| Bei-spiel Nr. | X | $Y^1$ | $Y^2$ | $Y^3$ | $Y^4$ | $R^1$ | 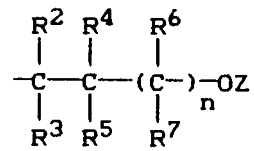 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 73 | 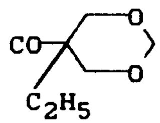 | H | Cl | Cl | H | H | $CH_2CH_2OCH_3$ | 71 |
| 74 | 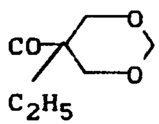 | H | Cl | Cl | H | H | $CH(CH_3)CH_2OCH_3$ | 50 |
| 75 | 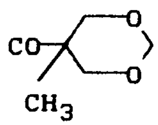 | H | Cl | Cl | H | H | $CH(CH_3)CH_2OCH_3$ | 61 |
| 76 | 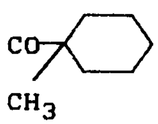 | H | Cl | Cl | H | H | $CH_2C(CH_3)_2OCH_3$ | 129 |

| Beispiel Nr. | X | $Y^1$ | $Y^2$ | $Y^3$ | $Y^4$ | $R^1$ | 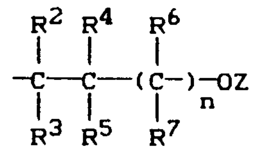 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 77 | 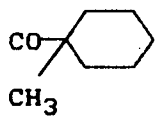 | H | Cl | H | H | H | $CHC(CH_3)_2OCH_3$ | |
| 78 | 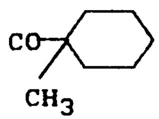 | H | Cl | Cl | H | H | $C(CH_3)_2CH_2OCH_3$ | |
| 79 | 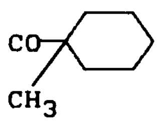 | H | Cl | H | H | H | $C(CH_3)_2CH_2OCH_3$ | |
| 80 | 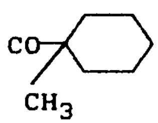 | H | Cl | Cl | H | H | 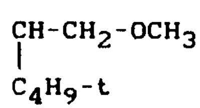 | |

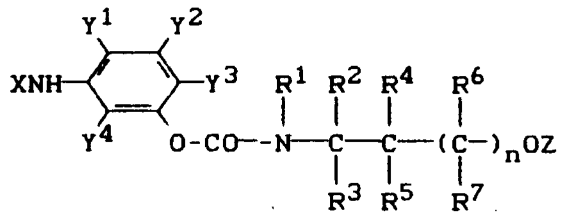

| Beispiel Nr. | X | $Y^1$ | $Y^2$ | $Y^3$ | $Y^4$ | $R^1$ | 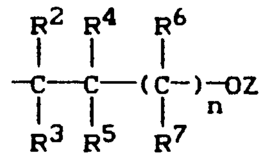 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 81 | 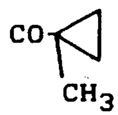 | F | H | Cl | H | H | $CH_2CH_2OCH_2CH_2OCH_3$ | 1,5322 |
| 82 | 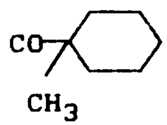 | F | H | $CH_3$ | H | H | $CH(C_2H_5)CH_2OCH_3$ | 70 |
| 83 | 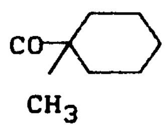 | F | H | $CH_3$ | H | H | $CH_2CH_2OCH_3$ | 102 |

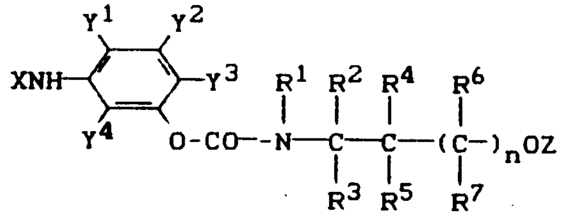

| Bei-spiel Nr. | X | $Y^1$ | $Y^2$ | $Y^3$ | $Y^4$ | $R^1$ | 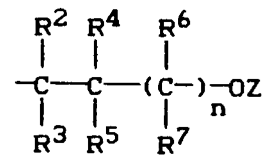 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 84 | 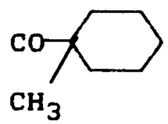 | F | H | Cl | H | H | $CH(CH_3)CH_2OCH_3$ | 85 |
| 85 | 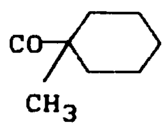 | F | H | Cl | H | H | $CH_2CH_2OCH_3$ | 110 |
| 86 | 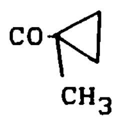 | F | H | Cl | H | H | $CH_2CH_2OCH_3$ | 97 |

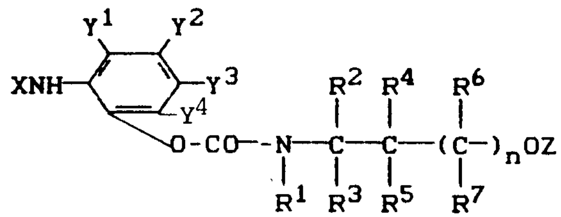

| Beispiel Nr. | X | $Y^1$ | $Y^2$ | $Y^3$ | $Y^4$ | $R^1$ | 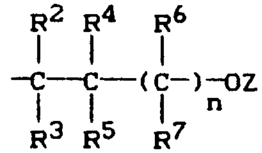 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 87 | 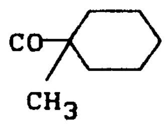 | H | Cl | H | H | H | $CH_2CH_2OCH_3$ | 94 |
| 88 | 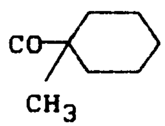 | H | H | H | H | H | $CH_2CH_2OCH_3$ | 101 |
| 89 | 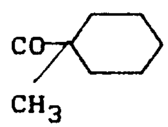 | H | Cl | H | Cl | H | $CH_2CH_2OCH_3$ | 146 |

| Beispiel Nr. | X | $Y^1$ | $Y^2$ | $Y^3$ | $Y^4$ | $R^1$ | 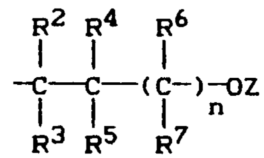 | Physikalische Daten: * Rf-Wert; Kieselgel 60F254; (Merck)-Laufmittel; Toluol:Aceton = 7:3; Brechungsindex $n_D^{20}$; Schmelzpunkt °C |
|---|---|---|---|---|---|---|---|---|
| 90 | $COC(CH_3)_3$ | H | $C(CH_3)_3$ | H | H | H | $CH_2CH_2OCH_3$ | 132 |
| 91 | $COC(CH_3)_3$ | H | F | H | H | H | $CH_2CH_2OCH_3$ | 104 |
| 92 | $COC(CH_3)_3$ | H | $SO_2CH_3$ | H | H | H | $CH_2CH_2OCH_3$ | *0,35 |
| 93 | 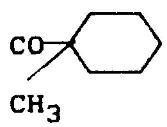 | H | Cl | H | H | H | $CH_2CH_2OCH_3$ | 101 |

Herstellung der Ausgangsprodukte der Formel (III)

Beispiel III

1. 2-Methoxy-ethylisocyanat

1.1 Herstellung von N-(2-Methoxyethyl)-carbamid-säure-2-ethylhexyl)-ester
(entsprechend der EP-PS 0 027 940

In einem 4 l Rundkolben mit Rückflußkühler werden 225 g (3,0 mol) 2-Methoxyethylamin, 180 g (3,0 mol) Harnstoff, 1950 g (15 mol) 2-Ethylhexanol und 3 g Dibutylzinnoxid vorgelegt und unter Rühren erhitzt. Bei Temperaturen oberhalb 110°C setzt eine lebhafte Ammoniak-Entwicklung ein. Ammoniak-Gas wird über den Rückflußkühler abgeleitet und in Wasser absorbiert. Im Verlaufe von 3 Stunden wird die Temperatur auf 180°C (Rückfluß) erhöht, wobei die Ammoniak-Abspaltung praktisch zum Stillstand kam. Nach weiteren 2 Stunden am Rückfluß wird das Gemisch durch Spülen mit Stickstoff von Restgehalten an Ammoniak befreit und anschließend fraktioniert destilliert. Nach einem hauptsächlich aus Ethylhexanol bestehenden Vorlauf destilliert das gesuchte Urethan bei 98 - 100°C/0,1 mbar als farblose Flüssigkeit über (Ausbeute: 617 g = 89 % der Theorie). Als Destillationsrückstand bleiben 27 g braune Flüssigkeit zurück, die beim Abkühlen pastös erstarrt.

1.2 Thermische Spaltung von N-(2-Methoxyethyl)-carbamidsäure-(2-ethylhexyl)-ester
(entsprechend der EP 0 054 817)

Als Spaltungsapparatur dient ein mit Tropftrichter und Rührer versehener Rundkolben, auf den zwei übereinander angeordnete Rückflußkühler aufgesetzt werden, die mittels Wärmeträgeröl thermostatisiert werden. Zwischen den beiden Rückflußkühlern ist ein Entnahmeboden angebracht.

Das Urethan wird kontinuierlich ohne Rückstandsabtrennung gespalten.

| | |
|---|---|
| Druck: | 29 mbar |
| Temperatur: | Spaltungskolben: 174-178°C |
| | Ölvorlauf unterer Kühler: 135°C |
| | Ölvorlauf oberer Kühler: 65°C |
| mittlere Verweilzeit: | etwa 4 Stunden |

| Spaltungsfraktionen: | | |
|---|---|---|
| Entnahmeboden: | 70,1 Gew.-% | Ethylhexanol |
| Kopf des oberen Kühlers: | 92,5 Gew.% | Isocyanat |

Nach Beendigung der Spaltung werden die erhaltenen Gemische jeweils fraktioniert destilliert.

| Gesamtergebnis der Spaltung: | | |
|---|---|---|
| Urethan: | eingesetzt: | 3,11kg |
| | zurückgewonnen: | 0,84 kg |
| | umgesetzt: | 2,27 kg |

2-Methoxyethylisocyanat, destilliert: 920n g
(Kp. 124°C), Reinheit: 99,3 % (GC)
→ Selektivität: 92 %
2-Ethylhexanol: 1,23 kg
→ Selektivität: 96 %
Rückstand: 114 g

Herstellung der Ausgangsprodukte zur Herstellung der Verbindungen der Formel (II) und Verbindungen der Formel (II)

Beispiel A1: 3.5-Dichlor-2,6-difluor-4-hydroxybenzoesäure

In einer Rührapparatur werden 300 g Kaliumhydroxid, 600 ml Wasser, 15 g Tetrabutylammoniumchlorid und 135 g 3,5-Dichlor-2,4,6-trifluorbenzotrifluorid vorgelegt und dann für 5 Std. auf Rückfluß erhitzt. Nach Ende der Reaktion wird abgekühlt und durch Zutropfen von Salzsäure sauer gestellt. Das Festprodukt wird abgesaugt und im Vakuum getrocknet. Ausbeute: 93 g mit einem Schmelzpunkt 102-5°C.

Beispiel A2: 3-Chlor-2,5,6-trifluor-4-hydroxy-benzoesäure

Analog Beispiel A1 werden aus 400 g NaOH, 1200 ml Wasser, 15 g Tetraethylammoniumchlorid und 276 g 3-Chlor-tetrafluorbenzotrifluorid bei 6 stündigem Erhitzen unter Rückfluß 238 g Produkt erhalten mit einem Schmelzpunkt von 87-90 ° C.

Beispiel A3: 2,6-Dichlor-3,5-difluorphenol

50 g 3,5-Dichlor-2,6-difluor-4-hydroxy-benzoesäure und 10 ml Dimethylformamid werden vermischt und erhitzt. Bei 105-130 ° C entwickelt sich Kohlendioxid und man läßt bei dieser Temperatur ausreagieren. Anschließend rührt man 200 ml Toluol und danach 80 ml Wasser ein, trennt die Phasen, trocknet die organische Phase und destilliert anschließend. Man erhält 34 g des Produktes mit einem Siedepunkt von 87-8 ° C; $n_D^{20}$ : 1,5310.

Beispiel A4:

Analog Beispiel A3 erhält man 2-Chlor-3,5,6-trifluorphenol, mit einem Siedepunkt 68-70 ° C/20 mbar.

Beispiel A5: 2,6-Dichlor-3,5-difluor-4-nitro-phenyl

In 70 ml Essigsäure werden 20 g 2,6-Dichlor-3,5-difluorphenol vorgelegt und 8 g 98 %ige Salpetersäure zugetropft. Anschließend wird für 2 Std. bei Raumtemperatur nachgerührt, in 150 ml Dichlormethan aufgenommen und zweimal mit Wasser gewaschen. Nach Abdestillieren des Dichlormethans verbleiben 18 g Produkt. Nach GC-Analyse 94 %ig.

Beispiel A6: 2-Chlor-3,5,6-trifluor-4-nitrophenol

Analog Beispiel A5 werden durch Nitrierung aus 28 g 2-Chlor-3,5,6-trifluorphenol 25 g 2-Chlor-3,5,6-trifluor-4-nitrophenol erhalten mit einer Reinheit von 93 % und einem Schmelzpunkt von 107-109 ° C.

Beispiel A7: 2,6-Dichlor-3,5-difluor-4-amino-phenol

18 g 2,6-Dichlor-3,5-difluor-4-nitrophenol werden in 100 ml Methanol in Gegenwart von 1,5 g Raney-Nickel bei 25-45 ° C mit 30-50 bar Wasserstoff bis zum Ende der Wasserstoffaufnahme hydriert. Nach Filtration wird die Lösung unter vermindertem Druck von Lösungsmittel befreit. Es verbleiben 13 g Aminophenol (Gl-Reinheit 98.4 %); Fp. 151 ° C.

Beispiel A8: 2-Chlor-3,5,6-trifluor-4-amino-phenol

Analog Beispiel A7 werden aus 25 g 2-Chlor-3.5.6-trifluor-4-nitro-phenol in 120 ml Methanol und 2 g Raney-Nickel durch Hydrierung 20 g Aminophenol (GC-Reinheit 97 %) erhalten.

Beispiel A9: (2,4-Difluor-5-nitro)-phenyl-trifluorchlorethylether

150 g Kaliumfluorid in 350 ml Tetramethylensulfon vorgelegt und mit Xylol azeotrop entwässert. Dann fügt man 200 g (2,4-Dichlor-5-nitro)-phenyl-trifluorchlorethylether hinzu und erhitzt für 5 Std. auf 180 ° C, anschließend für weitere 5 h auf 190-195 ° C unter Feuchtigkeitsausschluß. Nach Abkühlen wird der Ansatz in 1 l Wasser eingerührt und mehrmals mit Toluol extrahiert. Die Toluolphase wird zweimal mit Wasser gewaschen und dann getrocknet. Durch Destillieren werden 89 g (2,4-Difluor-5-nitro)-phenyl-trifluorchlorethylether erhalten mit einem Siedepunkt von 133-37 ° C/16 mbar.

Beispiel A10: 2.4-Difluor-5-nitrophenol

Man legt 50 g (2.4-Difluor-5-nitro)-phenyl-trifluorchlor-ethylether und 25 ml Tetramethylensulfon vor, fügt 50 ml konz. Schwefelsäure und 0,5 g Eisen-III-chlorid hinzu. Dann erhitzt man für 3 Std. auf 100 ° C, kühlt ab, gibt 100 g Eis zu, um anschließend nochmals für 2 h auf Rückfluß zu erhitzen. Nach Einrühren in 250 ml Wasser wird das Produkt mehrmals mit Toluol extrahiert, die Toluolphase getrocknet und destilliert. Man erhält 24 g 2,4-Difluor-5-nitrophenol.

Siedepunkt: 160-2 ° C/24 mbar, F. 117-19 ° C.

Beispiel A11: 2,4-Difluor-5-amino-phenol

Man unterwirft 24 g 2,4-Difluor-5-nitrophenol, gelöst in 120 ml Methanol, in Gegenwart von 2 g Raney-Nickel bei 25-45 ° C einer Hydrierung mit einem Wasserstoffdruck von 20-40 bar. Nach Druckkonstanz (d.h. Ende der Wasserstoffaufnahme) wird entspannt und abgekühlt, die Lösung durch Filtration vom Katalysator befreit und anschließend das Methanol bei vermindertem Druck abdestillieren. Man erhält 18 g (GC-Reinheit 97,5 %) 2,4-Difluor-5-amino-phenol.

Beispiel A

Botrytis-Test (Bohne)/protektiv

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden auf jedes Blatt 2 kleine mit Botrytis cinerea bewachsene Agarstückchen aufgelegt. Die inokulierten Pflanzen werden in einer abgedunkelten, feuchten Kammer bei 20 ° C aufgestellt. 3 Tage nach der Inokulation wird die Größe der Befallsflecken auf den Blättern ausgewertet.

Die Verbindungen z.B. gemäß den Herstellungsbeispielen 39 und 42 zeigen bei 50 ppm einen Befallsgrad von 5 %.

Beispiel B

Pyricularia-Test (Reis) /protektiv

Lösungsmittel: 12,5 Gewichtsteile Aceton
Emulgator: 0,3 Gewichtsteile Alkylarylpolyglykolether

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. Nach dem Abtrocknen des Spritzbelages werden die Pflanzen mit einer wäßrigen Sporensuspension von Pyricularia oryzae inokuliert. Anschließend werden die Pflanzen in einem Gewächshaus bei 100 % rel. Luftfeuchtigkeit und 25 ° C aufgestellt.

4 Tage nach der Inokulation erfolgt die Auswertung des Krankheitsbefalls.

Die Verbindungen gemäß den Herstellungsbeispielen: 6, 10, 14, 15, 18, 19, 21, 23, 27 und 28 zeigen z.B. bei einer Wirkstoffkonzentratrion von 0,025 % einen Krankheitsbefall von 0 bis 10 %.

**Patentansprüche**
**Patentansprüche für folgende Vertragsstaaten : BE, CH, DE, FR, GB, IT, LI, NL**

**1.** Substituierte Aminophenyl-carbamate der Formel (I)

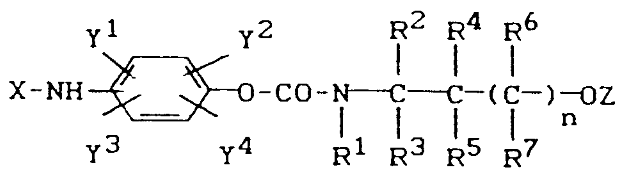

(I)

in welcher

X für $-CO-NR^8R^9$; $-CO-OR^9$; $CO-SR^9$; $-CO-R^{10}$; $-SO_2R^9$ oder Wasserstoff steht, worin

$R^8$ für Wasserstoff oder geradkettiges oder verzweightes Alkyl mit 1 bis 6 Kohlenstoffatomen steht,

$R^9$ für Aryl mit 6 bis 12 Kohlenstoffatomen, für ein- bis sechsfach, gleich oder verschieden durch Halogen, Alkyl mit 1 bis 6 Kohlenstoffatomen, Alkoxy mit 1 bis 6 Kohlenstoffatomen, Alkylthio mit 1 bis 6 Kohlenstoffatomen, Halogenalkyl mit 1 bis 6 Kohlenstoffatomen und 1 bis 9 gleichen oder verschiedenen Halogenatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Cyano und Nitro substituiertes Aryl mit 6 bis 12 Kohlenstoffatomen steht; ferner für Aralkyl mit 6 bis 12 Kohlenstoffatomen im Arylteil und mit 1 bis 6 Kohlenstoffatomen im geradkettigen oder verzweigten Alkylteil steht, wobei der Arylrest ein- bis sechsfach, gleich oder verschieden durch die obengenannten Arylsubstituenten substituiert sein kann, ferner für geradkettiges oder verzweigtes Alkyl mit 1 bis 20 Kohlenstoffatomen steht; ferner für ein- bis vierfach, gleich oder verschieden durch Halogen, Alkoxy mit 1 bis 6 Kohlenstoffatomen und Alkylthio mit 1 bis 6 Kohlenstoffatomen substituiertes Alkyl mit 1 bis 20 Kohlenstoffatomen steht, wobei die Alkylteile jeweils geradkettig oder verzweigt sein können; weiterhin für geradkettiges oder verzweigtes Alkenyl mit 2 bis 16 Kohlenstoffatomen oder für ein- bis sechsfach, gleich oder verschieden durch Halogen substituiertes Alkenyl mit 2 bis 6 Kohlenstoffatomen steht; weiterhin für Cycloalkyl mit 3 bis 10 Kohlenstoffatomen oder für Cycloalkyl-alkyl mit 1 bis 10 Kohlenstoffatomen im geradkettigen oder verzweigten Alkylteil und 3 bis 10 Kohlenstoffatomen im Cycloalkylteil steht, wobei die cyclischen Teile jeweils unabhängig, gleich oder verschieden, ein- bis sechsfach substituiert sein können durch Halogen, geradkettiges oder verzweigtes Alkyl mit 1 bis 6 Kohlenstoffatomen und Halogenalkyl mit 1 bis 6 Kohlenstoffatomen und 1 bis 9 gleichen oder verschiedenen Halogenatomen oder $R^9$ weiterhin für Cycloalkenyl mit 5 bis 10 Kohlenstoffatomen steht, das gleich oder verschieden, ein- bis sechsfach substituiert sein kann durch geradkettiges oder verzweigtes Alkyl mit 1 bis 6 Kohlenstoffatomen;

$R^{10}$ die Bedeutung von $R^9$ hat oder für einen Heterocyclus mit 5 bis 7 Ringgliedern steht, wobei außer Kohlenstoff ein oder mehrere Heteroatome enthalten sind. Der Heterocyclus kann ein- bis sechsfach, gleich oder verschieden durch geradkettiges oder verzweigtes Alkyl mit 1 bis 6 Kohlenstoffatomen substituiert sein;

$Y^1$ bis $Y^4$ gleich oder verschieden sind und für Wasserstoff, Halogen, Nitro, Cyano, geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen, Alkoxy oder Halogenalkoxy, Alkylthio, Alkylsulfonyl oder Halogenalkylthio mit jeweils 1 bis 6 Kohlenstoffatomen im geradkettigen oder verzweigten Alkylteil und gegebenenfalls 1 bis 9 gleichen oder verschiedenen Halogenatomen stehen;

$R^1$ bis $R^7$ gleich oder verschieden sind und für Wasserstoff, geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen oder für Alkoxyalkyl mit 1 bis 6 Kohlenstoffatomen je geradkettigem oder verzweigtem Alkylteil stehen;

n für 0 oder 1 steht und

Z für geradkettiges oder verzweigtes Alkyl oder Halogenalkyl mit je 1 bis 6 Kohlenstoffatomen und gegebenenfalls 1 bis 9 gleichen oder verschiedenen Halogenatomen oder für den Rest

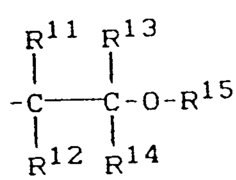

steht, worin

$R^{11}$ bis $R^{14}$ gleich oder verschieden sind und für Wasserstoff, für geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen oder Alkoxyalkyl mit 1 bis 6 Kohlenstoffatomen je geradkettigem oder verzweigtem Alkylteil stehen und

$R^{15}$ für geradkettiges oder verzweigtes Alkyl, Alkoxyalkyl oder Halogenalkyl mit je 1 bis 6 Kohlenstoffatomen je Alkylrest und gegebenenfalls 1 bis 9 gleichen oder verschiedenen Halogenatomen steht,

deren Diastereomeren und Diastereomerengemische.

**2.** Substituierte Aminophenyl-carbamate gemäß Anspruch 1, worin in der Formel (I)

X für $-CO-NR^8R^9$; $CO-OR^9$; $-CO-R^{10}$; $-SO_2R^9$ oder Wasserstoff steht, worin

$R^8$ für Wasserstoff steht,

$R^9$ für gegebenenfalls ein- bis dreifach, gleich oder verschieden im Phenylteil durch Halogen, geradkettig oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Halogenalkyl, Halogenalkoxy oder Halogenalkylthio mit jeweils 1 bis 4 Kohlenstoffatomen im geradkettigen oder verzweigten Alkylteil und 1 bis 5 gleichen oder verschiedenen Halogenatomen, Cyano oder Nitro jeweils substituiertes Phenyl, Benzyl, Phenylethyl oder 1-Phenyl-1-methyl-ethyl steht; für geradkettiges oder verzweigtes Alkyl mit 1 bis 18 Kohlenstoffatomen; für einbis dreifach, gleich oder verschieden durch Halogen, Alkoxy oder Alkylthio substituiertes Alkyl mit 1 bis 4 Kohlenstoffatomen im geradkettigen oder verzweigten Alkoxy- oder Alkylthioteil und 1 bis 10 Kohlenstoffatomen im Alkylteil; für geradkettiges oder verzweigtes Alkenyl mit 2 bis 8 Kohlenstoffatomen; für ein- bis dreifach, gleich oder verschieden durch Halogen substituiertes, geradkettiges oder verzweigtes Alkenyl mit 2 bis 4 Kohlenstoffatomen; für gegebenenfalls ein- bis dreifach, gleich oder verschieden durch Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 5 gleichen oder verschiedenen Halogenatomen jeweils substituiertes Cycloalkyl mit 3 bis 6 Kohlenstoffatomen oder Cycloalkyl-alkyl mit 3 bis 6 Kohlenstoffatomen im Cycloalkylteil und 1 bis 6 Kohlenstoffatomen im geradkettigen oder verzweigten Alkylteil steht;

$R^{10}$ die Bedeutung von $R^9$ hat oder für einen gegebenenfalls ein- bis dreifach durch geradkettiges oder verzweigtes Alkyl mit 1 bis 4 Kohlenstoffatomen substituierten 5- oder 6-gliedrigen Heterocyclus mit einem oder zwei Sauerstoffatomen steht,

$Y^1$ bis $Y^4$ gleich oder verschieden sind und für Wasserstoff, Halogen, geradkettiges oder verzweigtes Alkyl mit 1 bis 4 Kohlenstoffatomen, geradkettiges oder verzweigtes Alkoxy oder Alkylsulfonyl mit je 1 bis 4 Kohlenstoffatomen oder Halogenalkyl mit 1 bis 4 Kohlenstoffatomen im geradkettigen oder verzweigten Alkylteil und 1 bis 5 gleichen oder verschiedenen Halogenatomen stehen;

$R^1$ bis $R^7$ gleich oder verschieden sind und für Wasserstoff oder geradkettiges oder verzweigtes Alkyl mit 1 bis 6 Kohlenstoffatomen stehen,

n für 0 steht und

Z für geradkettiges oder verzweigtes Alkyl oder Halogenalkyl mit je 1 bis 4 Kohlenstoffatomen und gegebenenfalls 1 bis 5 gleichen oder verschiedenen Halogenatomen oder für den Rest

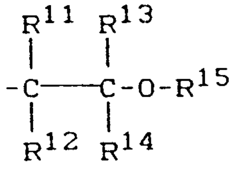

steht, worin

$R^{11}$ bis $R^{14}$ gleich oder verschieden sind und für Wasserstoff, geradkettiges oder verzweigtes Alkyl oder Halogenalkyl mit jeweils 1 bis 4 Kohlenstofatomen und gegebenenfalls 1 bis 5 gleichen oder verschiedenen Halogenatomen steht und

$R^{15}$ für geradkettiges oder verzweigtes Alkyl, Alkoxyalkyl oder Halogenalkyl mit jeweils 1 bis 4 Kohlenstoffatomen je Alkylrest und gegebenenfalls 1 bis 5 gleichen oder verschiedenen Halogenatomen steht,

deren Diastereomeren und Diastereomerengemische.

**3.** Substituierte Aminophenyl-carbamate gemäß Anspruch 1, worin in der Formel (I)

X für $-CO-NHR^9$; $-CO-OR^9$; $-CO-R^{10}$ oder $-SO_2R^9$ steht, worin

$R^9$ für gegebenenfalls ein- bis dreifach, gleich oder verschieden durch Methyl, Ethyl, Methoxy, Ethoxy, Trichlormethyl, Trifluormethyl, Trichlormethoxy, Trifluormethoxy, Fluor und Chlor substituiertes Phenyl steht; für Benzyl; für geradkettiges oder verzweigtes Alkyl mit 1 bis 5 Kohlenstoffatomen; für Halogenalkyl mit 1 bis 4 Kohlenstoffatomen und 1 oder 2 Chlor- oder Fluoratomen; für Halogenalkenyl mit 2 oder 3 Kohlenstoffatomen und 1 bis 4 Chloratomen; für gegebenenfalls jeweils ein- bis dreifach, gleich oder verschieden durch geradkettiges oder verzweigtes Alkyl mit 1 bis 4 Kohlenstoffatomen substituiertes Cyclopropyl oder Cyclohexyl steht,

$R^{10}$ für geradkettiges oder verzweigtes Alkyl mit 1 bis 18 Kohlenstoffatomen, für gegebenenfalls durch Methyl, Ethyl, n-Propyl, iso-Propyl, Chlor oder Trifluormethyl ein- bis dreifach, gleich oder verschieden substituiertes Phenyl, Cyclopropyl oder Cyclohexyl; gegebenenfalls ein- oder zweifach durch Chlor substituiertes Phenylalkyl mit 1 bis 3 Kohlenstoffatomen im geradkettigen oder verzweigten Alkylteil; für geradkettiges oder verzweigtes Halogenalkyl mit 1 bis 4 Kohlenstoffatomen und 1 oder 2 Chlor- oder Fluoratome; für geradkettiges oder verzweigtes Halogenalkenyl mit 2 bis 4 Kohlenstoffatomen und 1 bis 4 gleichen oder verschiedenen Halogenatomen, wie Chlor und Fluor, oder für gegebenenfalls einfach durch Methyl oder Ethyl substituiertes Oxanyl, Oxolanyl, Dioxanyl oder Dioxolanyl steht;

$Y^1$ bis $Y^4$ gleich oder verschieden sind und für Wasserstoff, Chlor, Fluor, Brom, Methyl, t-Butyl, Trifluormethyl oder Methylsulfonyl stehen,

$R^1$ bis $R^7$ gleich oder verschieden sind und für Wasserstoff oder Methyl stehen,

n für 0 steht und

Z für geradkettiges oder verzweigtes Alkyl mit 1 bis 4 Kohlenstoffatomen oder für den Rest

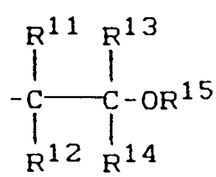

steht, worin

$R^{11}$ bis $R^{14}$ für Wasserstoff und

$R^{15}$ für Methyl, Ethyl, Propyl, Butyl, Methoxyethyl oder Ethoxyethyl steht,

deren Diastereomeren und Diastereomerengemische.

**4.** Verfahren zur Herstellung von substituierten Aminophenyl-carbamaten der Formel (I)

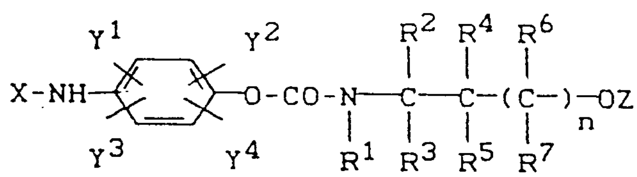

(I)

in welcher
n, X, $Y^1$-$Y^4$, Z und $R^1$-$R^7$ die in Anspruch 1 angegebenen Bedeutungen haben, dadurch gekennzeichnet, daß man Aminophenole der Formel (II)

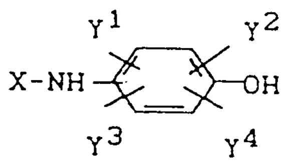

(II)

in welcher
X und $Y^1$ bis $Y^4$ die oben angegebenen Bedeutungen haben,
a) mit Isocyanaten der Formel (III)

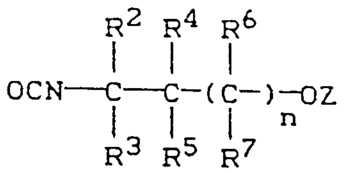

(III)

in welcher

$R^2$ bis $R^7$, n und Z die oben angegebenen Bedeutungen haben, gegebenenfalls in Gegenwart einer Base und gegebenenfalls in Gegenwart von Verdünnungs- oder Lösungsmitteln umsetzt, oder
b) mit Halogencarbonylverbindungen der Formel (IV)

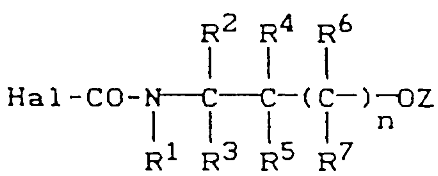

(IV)

in welcher

$R^1$ bis $R^7$, n und Z die oben angegebenen Bedeutungen haben, und

Hal für Halogen steht,

gegebenenfalls in Gegenwart eines Säureakzeptors und gegebenenfalls in Gegenwart eines Lösungs- oder Verdünnungsmittels umsetzt.

**5.** Verbindungen der Formel (I) gemäß den Ansprüchen 1 und 4, worin der gegebenenfalls substituierte Aminorest in 2, 3- oder 4-Stellung zum Carbamatrest steht.

**6.** Schädlingsbekämpfungsmittel, gekennzeichnet durch einen Gehalt an mindestens einem substituierten Aminophenyl-carbamat der Formel (I) nach den Ansprüchen 1 oder 4.

**7.** Verwendung von substituierten Aminophenylcarbamaten der Formel (I) nach den Ansprüchen 1 oder 4 zur Bekämpfung von Schädlingen.

**8.** Verfahren zur Bekämpfung von Schädlingen, dadurch gekennzeichnet, daß man substituierte Aminophenylcarbamate der Formel (I) nach den Ansprüchen 1 oder 4 auf Schädlinge und/oder ihren

Lebensraum einwirken läßt.

**9.** Verfahren zur Herstellung von Schädlingsbekämpfungsmitteln, dadurch gekennzeichnet, daß man substituierte Aminophenyl-carbamate der Formel (I) nach den Ansprüchen 1 oder 4 mit Streckmitteln und/oder oberflächenaktiven Mitteln vermischt.

**10.** Aminophenole der Formel (IIA) bzw. (IIB)

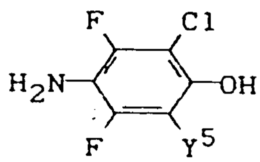 (IIA) bzw. 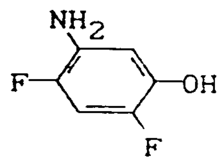 (IIB)

in welcher

$Y^5$ für Fluor oder Chlor steht.

**11.** Nitrophenole der Formeln (VIIA) bzw. (VIIB)

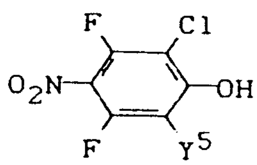 (VIIA) bzw. 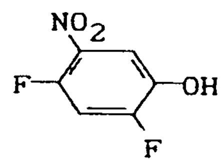 (VIIB)

in welcher
$Y^5$ für Fluor oder Chlor steht.

**12.** Verfahren zur Herstellung von Aminophenolen der Formeln (IIA) und (IIB)

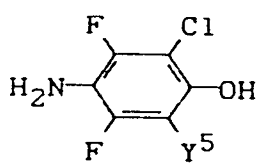 (IIA) bzw. 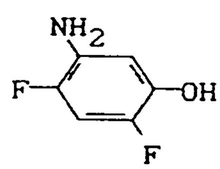 (IIB)

in welcher
$Y^5$ für Fluor oder Chlor steht,
dadurch gekennzeichnet, daß man Hydroxybenzoesäuren der Formeln (VA) bzw. (VB)

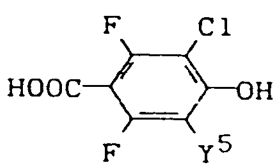 (VA) bzw. 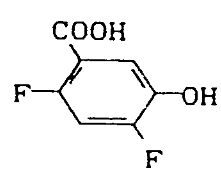 (VB)

decarboxyliert und die entstehenden Phenole der Formeln (VIA) bzw. (VIB)

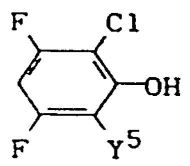 (VIA) bzw. 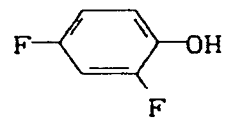 (VIB)

zu den Verbindungen der Formeln (VIIA) bzw. (VIIB) nitriert

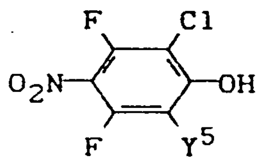 (VIIA) bzw. 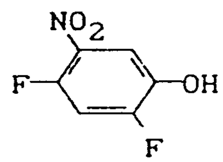 (VIIB)

in welcher
$Y^5$ für Fluor oder Chlor steht,
und diese anschließend hydriert.

**Patentansprüche für folgenden Vertragsstaat : ES**

**1.** Verfahren zur Herstellung von substituierten Aminophenyl-carbamaten der Formel (I)

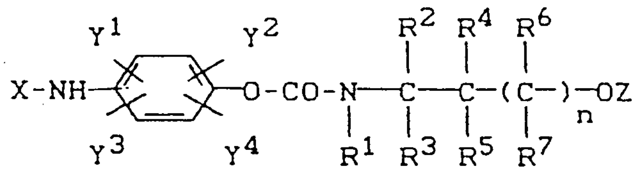 (I)

in welcher

X für $CO-NR^8R^9$; $-CO-OR^9$; $-CO-SR^9$; $-CO-R^{10}$; $-SO_2R^9$ oder Wasserstoff steht, worin

$R^8$ für Wasserstoff oder geradkettiges oder verzweigtes Alkyl mit 1 bis 6 Kohlenstoffatomen steht,

$R^9$ für Aryl mit 6 bis 12 Kohlenstoffatomen, für ein- bis sechsfach, gleich oder verschieden durch Halogen, Alkyl mit 1 bis 6 Kohlenstoffatomen, Alkoxy mit 1 bis 6 Kohlenstoffatomen, Alkylthio mit 1 bis 6 Kohlenstoffatomen, Halogenalkyl mit 1 bis 6 Kohlenstoffatomen und 1 bis 9 gleichen oder verschiedenen Halogenatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Cyano und Nitro substituiertes Aryl mit 6 bis 12 Kohlenstoffatomen steht; ferner für Aralkyl mit 6 bis 12 Kohlenstoffatomen im Arylteil und mit 1 bis 6 Kohlenstoffatomen im geradkettigen oder verzweigten Alkylteil steht, wobei der Arylrest ein- bis sechsfach, gleich oder verschieden durch die obengenannten Arylsubstituenten substituiert sein kann, ferner für geradkettiges oder verzweigtes Alkyl mit 1 bis 20 Kohlenstoffatomen steht; ferner für ein- bis vierfach, gleich oder verschieden durch Halogen, Alkoxy mit 1 bis 6 Kohlenstoffatomen und Alkylthio mit 1 bis 6 Kohlenstoffatomen substituiertes Alkyl mit 1 bis 20 Kohlenstoffatomen steht, wobei die Alkylteile jeweils geradkettig oder verzweigt sein können; weiterhin für geradkettiges oder verzweigtes Alkenyl mit 2 bis 16 Kohlenstoffatomen oder für ein- bis sechsfach, gleich oder verschieden durch Halogen substituiertes Alkenyl mit 2 bis 6 Kohlenstoffatomen steht; weiterhin für Cycloalkyl mit 3 bis 10 Kohlenstoffatomen oder für Cycloalkyl-alkyl mit 1 bis 10 Kohlenstoffatomen im geradkettigen oder verzweigten Alkylteil und 3 bis 10 Kohlenstoffatomen im Cycloalkylteil steht, wobei die cyclischen Teile jeweils unabhängig, gleich oder verschieden, ein- bis sechsfach substituiert sein können durch Halogen, geradkettiges oder verzweigtes Alkyl mit 1 bis 6 Kohlenstoffatomen und Halogenalkyl mit 1 bis 6 Kohlenstoffatomen und 1 bis 9 gleichen oder verschiedenen Halogenatomen oder $R^9$ weiterhin für Cycloalkenyl mit 5 bis 10 Kohlenstoffatomen steht, das gleich oder verschieden, ein- bis sechsfach substituiert sein kann durch geradkettiges oder verzweigtes Alkyl mit 1 bis 6 Kohlenstoffatomen;

$R^{10}$ die Bedeutung von $R^9$ hat oder für einen Heterocyclus mit 5 bis 7 Ringgliedern steht, wobei außer Kohlenstoff ein oder mehrere Heteroatome enthalten sind. Der Heterocyclus kann ein- bis sechsfach, gleich oder verschieden durch geradkettiges oder verzweigtes Alkyl mit 1 bis 6 Kohlenstoffatomen substituiert sein;

$Y^1$ bis $Y^4$ gleich oder verschieden sind und für Wasserstoff, Halogen, Nitro, Cyano, geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen, Alkoxy oder Halogenalkoxy, Alkylthio, Alkylsulfonyl oder Halogenalkylthio mit jeweils 1 bis 6 Kohlenstoffatomen im geradkettigen oder verzweigten Alkylteil und gegebenenfalls 1 bis 9 gleichen oder verschiedenen Halogenatomen stehen;

$R^1$ bis $R^7$ gleich oder verschieden sind und für Wasserstoff, geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen oder für Alkoxyalkyl mit 1 bis 6 Kohlenstoffatomen je geradkettigem oder verzweigtem Alkylteil stehen;

n für 0 oder 1 steht und

Z für geradkettiges oder verzweigtes Alkyl oder Halogenalkyl mit je 1 bis 6 Kohlenstoffatomen und gegebenenfalls 1 bis 9 gleichen oder verschiedenen Halogenatomen oder für den Rest

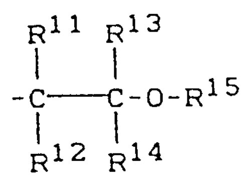

steht, worin

$R^{11}$ bis $R^{14}$ gleich oder verschieden sind und für Wasserstoff, für geradkettiges oder verzweigtes Alkyl mit 1 bis 6 Kohlenstoffatomen, Halogenalkyl mit 1 bis 6 Kohlenstoffatomen im geradkettigen oder verzweigten Alkylteil und 1 bis 9 gleichen oder verschiedenen Halogenatomen oder Alkoxyalkyl mit 1 bis 6 Kohlenstoffatomen je geradkettigem oder verzweigtem Alkylteil stehen und

$R^{15}$ für geradkettiges oder verzweigtes Alkyl, Alkoxyalkyl oder Halogenalkyl mit je 1 bis 6 Kohlenstoffatomen je Alkylrest und gegebenenfalls 1 bis 9 gleichen oder verschiedenen Halogenatomen steht,

deren Diastereomeren und Diastereomerengemische, dadurch gekennzeichnet, daß man Aminophenole der Formel (II)

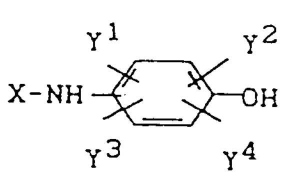

(II)

in welcher

X und $Y^1$ bis $Y^4$ die oben angegebenen Bedeutungen haben,

a) mit Isocyanaten der Formel (III)

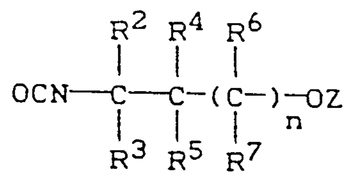

(III)

in welcher

$R^2$ bis $R^7$, n und Z die oben angegebenen Bedeutungen haben, gegebenenfalls in Gegenwart einer Base und gegebenenfalls in Gegenwart von Verdünnungs- oder Lösungsmitteln umsetzt, oder

b) mit Halogencarbonylverbindungen der Formel (IV)

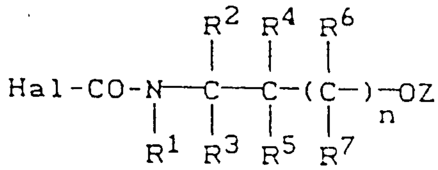

(IV)

in welcher

$R^1$ bis $R^7$, n und Z die oben angegebenen Bedeutungen haben, und

Hal für Halogen steht,

gegebenenfalls in Gegenwart eines Säureakzeptors und gegebenenfalls in Gegenwart eines Lösungs- oder Verdünnungsmittels umsetzt.

**2.** Verfahren gemäß Anspruch 1, worin in der Formel (I)

X für $-CO-NR^8R^9$; $-CO-OR^9$; $-CO-R^{10}$; $-SO_2R^9$ oder Wasserstoff steht, worin

$R^8$ für Wasserstoff steht,

$R^9$ für gegebenenfalls ein- bis dreifach, gleich oder verschieden im Phenylteil durch Halogen, geradkettig oder verzweigtes Alkyl mit 1 bis 4 Kohlenstoffatomen, geradkettiges oder verzweigtes Alkoxy mit 1 bis 4 Kohlenstoffatomen, geradkettiges oder verzweigtes Alkylthio mit 1 bis 4 Kohlenstoffatomen, Halogenalkyl, Halogenalkoxy oder Halogenalkylthio mit jeweils 1 bis 4 Kohlenstoffatomen im geradkettigen oder verzweigten Alkylteil und 1 bis 5 gleichen oder verschiedenen Halogenatomen, Cyano oder Nitro jeweils substituiertes Phenyl, Benzyl, Phenylethyl oder 1-Phenyl-1-methyl-ethyl steht; für geradkettiges oder verzweigtes Alkyl mit 1 bis 18 Kohlenstoffatomen; für einbis dreifach, gleich oder verschieden durch Halogen, Alkoxy oder Alkylthio substituiertes Alkyl mit 1 bis 4 Kohlenstoffatomen im geradkettigen oder verzweigten Alkoxy- oder Alkylthioteil und 1 bis 10 Kohlenstoffatomen im Alkylteil; für geradkettiges oder verzweigtes Alkenyl mit 2 bis 8 Kohlenstoffatomen; für ein- bis dreifach, gleich oder verschieden durch Halogen substituiertes, geradkettiges oder verzweigtes Alkenyl mit 2 bis 4 Kohlenstoffatomen; für gegebenenfalls ein- bis dreifach, gleich oder verschieden durch Halogen, geradkettiges oder verzweigtes Alkyl mit 1 bis 4 Kohlenstoffatomen oder geradkettiges oder verzweigtes Halogenalkyl mit 1 bis 4 Kohlenstoffatomen und 1 bis 5 gleichen oder verschiedenen Halogenatomen jeweils substituiertes Cycloalkyl mit 3 bis 6 Kohlenstoffatomen oder Cycloalkyl-alkyl mit 3 bis 6 Kohlenstoffatomen im Cycloalkylteil und 1 bis 6 Kohlenstoffatomen im geradkettigen oder verzweigten Alkylteil steht;

$R^{10}$ die Bedeutung von $R^9$ hat oder für einen gegebenenfalls ein- bis dreifach durch geradkettiges oder verzweigtes Alkyl mit 1 bis 4 Kohlenstoffatomen substituierten 5-

oder 6-gliedrigen Heterocyclus mit einem oder zwei Sauerstoffatomen steht,

$Y^1$ bis $Y^4$ gleich oder verschieden sind und für Wasserstoff, Halogen, geradkettiges oder verzweigtes Alkyl mit 1 bis 4 Kohlenstoffatomen, geradkettiges oder verzweigtes Alkoxy oder Alkylsulfonyl mit je 1 bis 4 Kohlenstoffatomen oder Halogenalkyl mit 1 bis 4 Kohlenstoffatomen im geradkettigen oder verzweigten Alkylteil und 1 bis 5 gleichen oder verschiedenen Halogenatomen stehen;

$R^1$ bis $R^7$ gleich oder verschieden sind und für Wasserstoff oder geradkettiges oder verzweigtes Alkyl mit 1 bis 6 Kohlenstoffatomen stehen,

n für 0 steht und

Z für geradkettiges oder verzweigtes Alkyl oder Halogenalkyl mit je 1 bis 4 Kohlenstoffatomen und gegebenenfalls 1 bis 5 gleichen oder verschiedenen Halogenatomen oder für den Rest

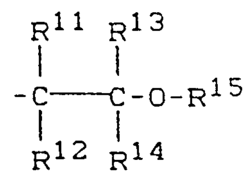

steht, worin

$R^{11}$ bis $R^{14}$ gleich oder verschieden sind und für Wasserstoff, geradkettiges oder verzweigtes Alkyl oder Halogenalkyl mit jeweils 1 bis 4 Kohlenstofatomen und gegebenenfalls 1 bis 5 gleichen oder verschiedenen Halogenatomen steht und

$R^{15}$ für geradkettiges oder verzweigtes Alkyl, Alkoxyalkyl oder Halogenalkyl mit jeweils 1 bis 4 Kohlenstoffatomen je Alkylrest und gegebenenfalls 1 bis 5 gleichen oder verschiedenen Halogenatomen steht,

deren Diastereomeren und Diastereomerengemische.

**3.** Verfahren gemäß Anspruch 1, worin in der Formel (I)

X für $-CO-NHR^9$; $-CO-OR^9$; $CO-R^{10}$ oder $-SO_2R^9$ steht, worin

$R^9$ für gegebenenfalls ein- bis dreifach, gleich oder verschieden durch Methyl, Ethyl, Methoxy, Ethoxy, Trichlormethyl, Trifluormethyl, Trichlormethoxy, Trifluormethoxy, Fluor und Chlor substituiertes Phenyl steht; für Benzyl; für geradkettiges oder verzweigtes Alkyl mit 1 bis 5 Kohlenstoffatomen; für Halogenalkyl mit 1 bis 4 Kohlenstoffatomen und 1 oder 2 Chlor- oder Fluoratomen; für Halogenalkenyl mit 2 oder 3 Kohlenstoffatomen und 1 bis 4 Chloratomen; für gegebenenfalls jeweils ein- bis dreifach, gleich oder verschieden durch geradkettiges oder verzweigtes Alkyl mit 1 bis 4 Kohlenstoffatomen substituiertes Cyclopropyl oder Cyclohexyl steht,

$R^{10}$ für geradkettiges oder verzweigtes Alkyl mit 1 bis 18 Kohlenstoffatomen, für gegebenenfalls durch Methyl, Ethyl, n-Propyl, iso-Propyl, Chlor oder Trifluormethyl ein- bis dreifach, gleich oder verschieden substituiertes Phenyl, Cyclopropyl oder Cyclohexyl; gegebenenfalls ein- oder zweifach durch Chlor substituiertes Phenylalkyl mit 1 bis 3 Kohlenstoffatomen im geradkettigen oder verzweigten Alkylteil; für geradkettiges oder verzweigtes Halogenalkyl mit 1 bis 4 Kohlenstoffatomen und 1 oder 2 Chlor- oder Fluoratome; für geradkettiges oder verzweigtes Halogenalkenyl mit 2 bis 4 Kohlenstoffatomen und 1 bis 4 gleichen oder verschiedenen Halogenatomen, wie Chlor und Fluor, oder für gegebenenfalls einfach durch Methyl oder Ethyl substituiertes Oxanyl, Oxolanyl, Dioxanyl oder Dioxolanyl steht;

$Y^1$ bis $Y^4$ gleich oder verschieden sind und für Wasserstoff, Chlor, Fluor, Brom, Methyl, t-Butyl, Trifluormethyl oder Methylsulfonyl stehen,

$R^1$ bis $R^7$ gleich oder verschieden sind und für Wasserstoff oder Methyl stehen,

n für 0 steht und

Z für geradkettiges oder verzweigtes Alkyl mit 1 bis 4 Kohlenstoffatomen oder für den Rest

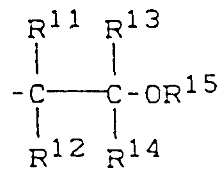

steht, worin

$R^{11}$ bis $R^{14}$ für Wasserstoff und

$R^{15}$ für Methyl, Ethyl, Propyl, Butyl, Methoxyethyl oder Ethoxyethyl steht,

deren Diastereomeren und Diastereomerengemische.

4. Verfahren gemäß den Ansprüchen 1 bis 3, worin der gegebenenfalls substituierte Aminorest in 2-, 3- oder 4-Stellung zum Carbamatrest steht.

5. Schädlinasbekämpfungsmittel, gekennzeichnet durch einen Gehalt an mindestens einem substituierten Aminophenyl-carbamat der Formel (I) nach den Ansprüchen 1 bis 4.

6. Verwendung von substituierten Aminophenylcarbamaten der Formel (I) nach den Ansprüchen 1 bis 4 zur Bekämpfung von Schädlingen.

7. Verfahren zur Bekämpfung von Schädlingen dadurch gekennzeichnet, daß man substituierte Aminophenylcarbamate der Formel (I) nach den Ansprüchen 1 bis 4 auf Schädlinge und/oder ihren Lebensraum einwirken läßt.

8. Verfahren zur Herstellung von Schädlingsbekämpfungsmitteln, dadurch gekennzeichnet, daß man substituierte Aminophenyl-carbamate der Formel (I) nach den Ansprüchen 1 bis 4 mit Streckmitteln und/oder oberflächenaktiven Mitteln vermischt.

9. Verfahren zur Herstellung von Aminophenolen der Formeln (IIA) und (IIB)

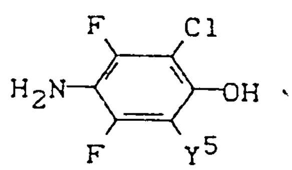 (IIA) bzw. 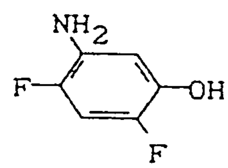 (IIB)

in welcher

$Y^5$ für Fluor oder Chlor steht,

dadurch gekennzeichnet, daß man Hydroxybenzoesäuren der Formeln (VA) bzw. (VB)

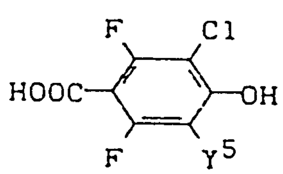 (VA) bzw. 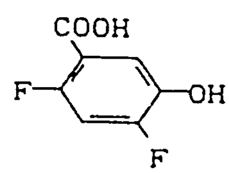 (VB)

decarboxyliert und die entstehenden Phenole der Formeln (VIA) bzw. (VIB)

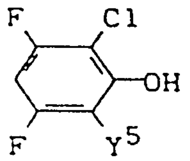 (VIA) bzw. 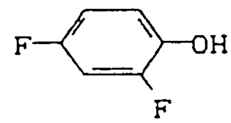 (VIB)

zu den Verbindungen der Formeln (VIIA) bzw. (VIIB) nitriert

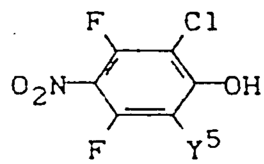 (VIIA) bzw. 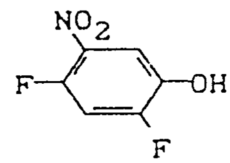 (VIIB)

in welcher

$Y^5$ für Fluor oder Chlor steht,

und diese anschließend hydriert.

**Claims**

**Claims for the following Contracting States : BE, CH, DE, FR, GB, IT, LI, NL**

**1.** Substituted aminophenyl carbamates of the formula (I) in which

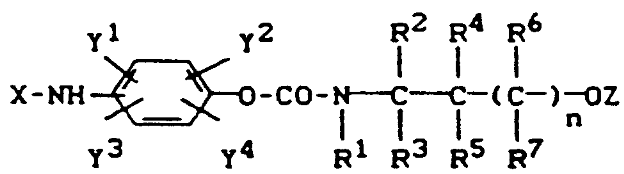 (I)

X represents -CO-NR$^8$R$^9$; -CO-OR$^9$; -CO-SR$^9$; CO-R$^{10}$; -SO$_2$R$^9$ or hydrogen, in which

$R^8$ represents hydrogen or straight-chain or branched alkyl having 1 to 6 carbon atoms,

$R^9$ represents aryl having 6 to 12 carbon atoms, aryl having 6 to 12 carbon atoms which is monosubstituted to hexasubstituted by identical or different substituents from the series comprising halogen, alkyl having 1 to 6 carbon atoms, alkoxy having 1 to 6 carbon atoms, alkylthio having 1 to 6 carbon atoms, halogenoalkyl having 1 to 6 carbon atoms and 1 to 9 identical or different halogen atoms, halogenoalkoxy having 1 to 6 carbon atoms and 1 to 9 identical or different halogen atoms, halogenoalkylthio having 1 to 6 carbon atoms and 1 to 9 identical or different halogen atoms, cyano and nitro; furthermore represents aralkyl having 6 to 12 carbon atoms in the aryl part and having 1 to 6 carbon atoms in the straight-chain or branched alkyl part, it being possible for the aryl radical to be monosubstituted to hexasubstituted by identical or different substituents from the series comprising the abovementioned aryl substituents; furthermore represents straight-chain or branched alkyl having 1 to 20 carbon atoms; furthermore represents alkyl having 1 to 20 carbon atoms which is monosubstituted to tetrasubstituted by identical or different substituents from the series comprising halogen, alkoxy having 1 to 6 carbon atoms and alkylthio having 1 to 6 carbon atoms, it being possible for each of the alkyl parts to be straight-chain or branched; in addition represents straight-chain or branched alkenyl having 2 to 16 carbon atoms or alkenyl having 2 to 6 carbon atoms which is monosubstituted to hexasubstituted by identical or different halogen; in addition represents cycloalkyl having 3 to 10 carbon atoms or cycloalkylalkyl having 1 to 10 carbon atoms in the straight-chain or branched alkyl part and 3 to 10 carbon atoms in the cycloalkyl part, it being possible for the cyclic parts, in each case independently, to be monosubstituted to hexasubstituted by identical or different substituents from the series comprising halogen, straight-chain or branched alkyl having 1 to 6 carbon atoms and halogenoalkyl having 1 to 6 carbon atoms and 1 to 9 identical or different halogen atoms, or $R^9$, in addition, represents cycloalkenyl which has 5 to 10 carbon atoms and which may be monosubstituted to hexasubstituted by identical or different, straight-chain or branched alkyl having 1 to 6 carbon atoms;

$R^{10}$ has the meaning of $R^9$ or represents a heterocyclic ring having 5 to 7 ring members, one or more hetero atoms being included in addition to carbon. The heterocyclic ring may be monosubstituted to hexasubstituted by identical or different, straight-chain or branched alkyl having 1 to 6 carbon atoms;

$Y^1$ to $Y^4$ are identical or different and represent hydrogen, halogen, nitro, cyano, straight-chain or branched alkyl having 1 to 6 carbon atoms, halogenoalkyl having 1 to 6 carbon atoms in the straight-chain or branched alkyl part and 1 to 9 identical or different halogen atoms, alkoxy or halogenoalkoxy, alkylthio, alkylsulphonyl or halogenoalkylthio in each case having 1 to 6 carbon atoms in the straight-chain or branched alkyl part and, if appropriate, 1 to 9 identical or different halogen atoms;

$R^1$ to $R^7$ are identical or different and represent hydrogen, straight-chain or branched alkyl having 1 :l to 6 carbon atoms, halogenoalkyl having 1 to 6 carbon atoms in the straight-chain or branched alkyl part and 1 to 9 identical or different halogen atoms, or alkoxyalkyl having 1 to 6 carbon atoms in each straight-chain or branched alkyl part;

n represents 0 or 1, and

Z represents straight-chain or branched alkyl or halogenoalkyl each having 1 to 6 carbon atoms and, if appropriate, 1 to 9 identical or different halogen atoms, or the

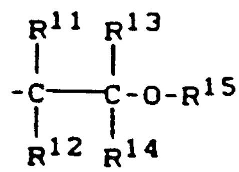

radical, in which

$R^{11}$ to $R^{14}$ are identical or different and represent hydrogen, straight-chain or branched alkyl having 1 to 6 carbon atoms, halogenoalkyl having 1 to 6 carbon atoms in the straight-chain or branched alkyl part and 1 to 9 identical or different halogen atoms, or alkoxyalkyl having 1 to 6 carbon atoms in each straight-chain or branched alkyl part, and

$R^{15}$ represents straight-chain or branched alkyl, alkoxyalkyl, or halogenoalkyl each having 1 to 6 carbon atoms per alkyl radical and, if appropriate, 1 to 9 identical or different halogen atoms,
and the diastereomers and diastereomer mixtures thereof.

**2.** Substituted aminophenyl carbamates according to Claim 1, in which, in the formula (I),

X represents $-CO-NR^8R^9$; $-CO-OR^9$; $-CO-R^{10}$; $-SO_2R^9$ or hydrogen, in which

$R^8$ represents hydrogen,

$R^9$ represents phenyl, benzyl, phenylethyl or 1-phenyl-1-methyl-ethyl each of which is optionally monosubstituted, disubstituted or trisubstituted in the phenyl part by identical or different substituents from the series comprising halogen, straight-chain or branched alkyl having 1 to 4 carbon atoms, straight-chain or branched alkoxy having 1 to 4 carbon atoms, straight-chain or branched alkylthio having 1 to 4 carbon atoms, halogenoalkyl, halogenoalkoxy or halogenoalkylthio in each case having 1 to 4 carbon in the straight-chain or branched alkyl part and 1 to 5 identical or different halogen atoms, cyano or nitro; represents straight-chain or branched alkyl having 1 to 18 carbon atoms; alkyl which has 1 to 4 carbon atoms in the straight-chain or branched alkoxy or alkylthio part and 1 to 10 carbon atoms in the alkyl part and which is monosubstituted, disubstituted or trisubstituted by identical or different substituents from the series comprising halogen, alkoxy or alkylthio; straight-chain or branched alkenyl having 2 to 8 carbon atoms; straight-chain or branched alkenyl having 2 to 4 carbon atoms which is monosubstituted, disubstituted or trisubstituted by identical or different halogen; cycloalkyl which has 3 to 6 carbon atoms or cycloalkyl-alkyl which has 3 to 6 carbon atoms in the cycloalkyl part and 1 to 6 carbon atoms in the straight-chain or branched alkyl part and each of which is optionally monosubstituted, disubstituted or trisubstituted by identical or different substituents from the series comprising halogen, straight-chain or branched alkyl having 1 to 4 carbon atoms or straight-chain or branched halogenoalkyl having 1 to 4 carbon atoms and 1 to 5 identical or different halogen atoms;

$R^{10}$ has the meaning of $R^9$ or represents a 5- or 6-membered heterocyclic ring which contains one or two oxygen atoms and which is optionally monosubstituted, disubstituted or trisubstituted by straight-chain or branched alkyl having 1 to 4 carbon atoms,

$Y^1$ to $Y^4$ are identical or different and represent hydrogen, halogen, straight-chain or branched alkyl having 1 to 4 carbon atoms, straight-chain or branched alkoxy or alkylsulphonyl each having 1 to 4 carbon atoms, or halogenoalkyl having 1 to 4 carbon atoms in the straight-chain or branched alkyl part and 1 to 5 identical or different halogen atoms;

$R^1$ to $R^7$ are identical or different and represent hydrogen or straight-chain or branched alkyl having 1 to 6 carbon atoms,

n represents 0, and

Z represents straight-chain or branched alkyl or halogenoalkyl each having 1 to 4 carbon atoms and, if appropriate, 1 to 5 identical or different halogen atoms, or the formula

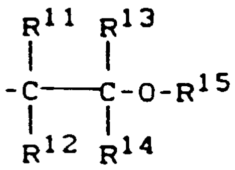

radical, in which

$R^{11}$ to $R^{14}$ are identical or different and represent hydrogen, straight-chain or branched alkyl or halogenoalkyl in each case having 1 to 4 carbon atoms and, if appropriate, 1 to 5 identical or different halogen atoms and

$R^{15}$ represents straight-chain or branched alkyl, alkoxyalkyl or halogenoalkyl in each case having 1 to 4 carbon atoms per alkyl radical and, if appropriate, 1 to 5 identical or different halogen atoms, and the diastereomers and diastereomer mixtures thereof.

**3.** Substituted aminophenyl carbamates according to Claim 1, in which, in the formula (I),

X represents $-CO-NHR^9$; $-CO-OR^9$; $-CO-R^{10}$ or $-SO_2R^9$ in which

$R^9$ represents phenyl which is optionally mono-substituted, disubstituted or trisubstituted by identical or different substituents from the series comprising methyl, ethyl, methoxy, ethoxy, trichloromethyl, trifluoromethyl, trichloromethoxy, trifluoromethoxy, fluorine and chlorine; represents benzyl; straight-chain or branched alkyl having 1 to 5 carbon atoms; halogenoalkyl having 1 to 4 carbon atoms and 1 or 2 chlorine or fluorine atoms; halogenoalkenyl having 2 or 3 carbon atoms and 1 to 4 chlorine atoms; cyclopropyl or cyclohexyl each of which is optionally monosubstituted, disubstituted or trisubstituted by identical or different, straight-chain or branched alkyl having 1 to 4 carbon atoms,

$R^{10}$ represents straight-chain or branched alkyl having 1 to 18 carbon atoms, represents phenyl, cyclopropyl or cyclohexyl each of which is optionally monosubstituted, disubstituted or trisubstituted by identical or different substituents from the series comprising methyl, ethyl, n-propyl, iso-propyl, chlorine or trifluoromethyl; phenylalkyl which has 1 to 3 carbon atoms in the straight-chain or branched alkyl part and which is optionally monosubstituted or disubstituted by chlorine; straight-chain or branched halogenoalkyl having 1 to 4 carbon atoms and 1 or 2 chlorine or fluorine atoms; straight-chain or branched halogenoalkenyl having 2 to 4 carbon atoms and 1 to 4 identical or different halogen atoms, such as chlorine and fluorine, or oxanyl, oxolanyl, dioxanyl or dioxolanyl each of which is optionally monosubstituted by methyl or ethyl;

$Y^1$ to $Y^4$ are identical or different and represent hydrogen, chlorine, fluorine, bromine, methyl, t-butyl, trifluoromethyl or methylsulphonyl,

$R^1$ to $R^7$ are identical or different and represent hydrogen or methyl,

n represents 0, and

Z represents straight-chain or branched alkyl having 1 to 4 carbon atoms, or the

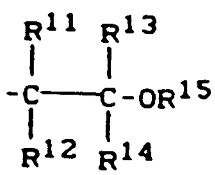

radical in which

$R^{11}$ to $R^{14}$ represent hydrogen, and

$R^{15}$ represents methyl, ethyl, propyl, butyl, methoxyethyl or ethoxyethyl, and the diastereomers and diastereomer mixtures thereof.

**4.** Process for the preparation of substituted aminophenyl carbamates of the formula (I)

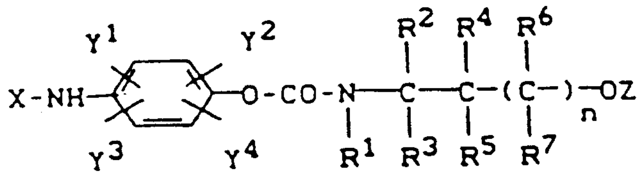

(I)

in which

n, X, $Y^1$-$Y^4$, Z and $R^1$-$R^7$ have the meaning mentioned in Claim 1, characterized in that aminophenols of the formula (II)

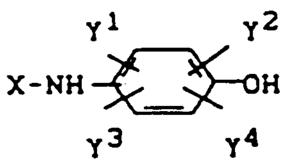

(II)

in which

X and $Y^1$ to $Y^4$ have the abovementioned meanings,

a) are reacted with isocyanates of the formula (III)

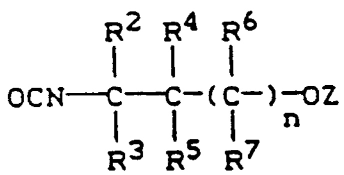

(III)

in which

$R^2$ to $R^7$, n and Z have the abovementioned meanings, if appropriate in the presence of a base and if appropriate in the presence of diluents or solvent, or

b) are reacted with halogenocarbonyl compounds of the formula (IV)

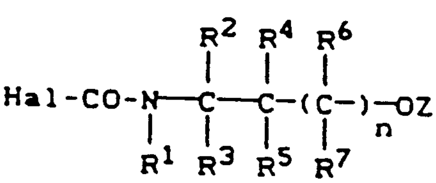

(IV)

in which

$R^1$ to $R^7$, n and Z have the abovementioned meanings, and

Hal represents halogen,

if appropriate in the presence of an acid acceptor and if appropriate in the presence of a solvent or diluent.

**5.** Compounds of the formula (I) according to Claims 1 and 4, in which the optionally substituted amino radical is in the 2- or 4-position to the carbamate radical.

**6.** Pesticides, characterized in that they contain at least one substituted aminophenyl carbamate of the

formula (I) according to Claims 1 or 4.

**7.** Use of substituted aminophenyl carbamates of the formula (I) according to Claims 1 or 4 for combating pests.

**8.** Process for combating pests, characterized in that substituted aminophenyl carbamates of the formula (I) according to Claims 1 or 4 are allowed to act on the pests and/or their environment.

**9.** Process for the production of pesticides, characterized in that substituted aminophenyl carbamates of the formula (I) according to Claims 1 or 4 are mixed with extenders and/or surface-active agents.

**10.** Aminophenols of the formula (IIA) or (IIB)

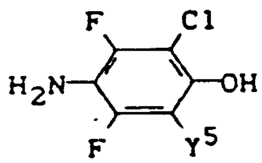 (IIA) and 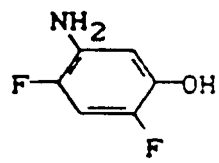 (IIB)

in which
$Y^5$ represents fluorine or chlorine.

**11.** Nitrophenols of the formulae (VIIA) and (VIIB)

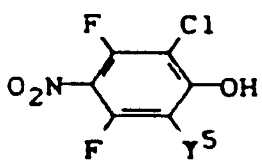 (VIIA) and 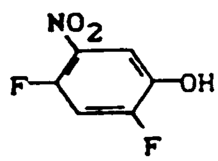 (VIIB)

in which
$Y^5$ represents fluorine or chlorine.

**12.** Process for the preparation of aminophenols of the formulae (IIA) and (IIB)

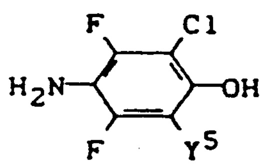 (IIA) and 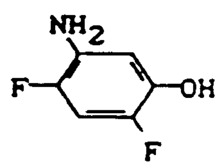 (IIB)

in which
$Y^5$ represents fluorine or chlorine, characterized in that hydroxybenzoic acids of the formulae (VA) and (VB)

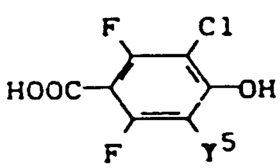 (VA) and 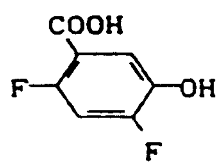 (VB)

are decarboxylated and the resultant phenols of the formulae (VIA) and (VIB) respectively

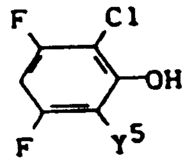 (VIA) and 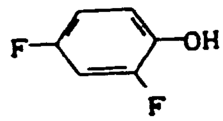 (VIB)

are nitrated to give the compounds of the formulae (VIIA) and (VIIB)

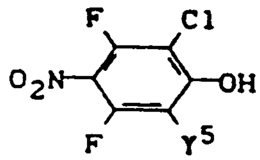 (VIIA) and 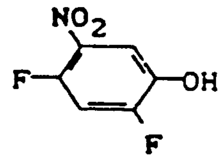 (VIIB)

in which

$Y^5$ represents fluorine or chlorine, and the latter are subsequently hydrogenated.

**Claims for the following Contracting State : ES**

1. Process for the preparation of substituted aminophenyl carbamates of the formula (I)

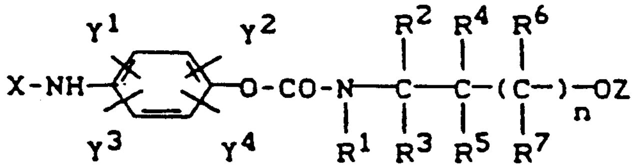 (I)

in which

X represents $-CO-NR^8R^9$; $-CO-OR^9$; $-CO-SR^9$; $-CO-R^{10}$; $-SO_2R^9$ or hydrogen, in which

$R^8$ represents hydrogen or straight-chain or branched alkyl having 1 to 6 carbon atoms,

$R^9$ represents aryl having 6 to 12 carbon atoms, aryl having 6 to 12 carbon atoms which is monosubstituted to hexasubstituted by identical or different substituents from the series comprising halogen, alkyl having 1 to 6 carbon atoms, alkoxy having 1 to 6 carbon atoms, alkylthio having 1 to 6 carbon atoms, halogenoalkyl having 1 to 6 carbon atoms and 1 to 9 identical or different halogen atoms, halogenoalkoxy having 1 to 6 carbon atoms and 1 to 9 identical or different halogen atoms, halogenoalkylthio having 1 to 6 carbon atoms and 1 to 9 identical or different halogen atoms, cyano and nitro; furthermore represents aralkyl having 6 to 12 carbon atoms in the aryl part and having 1 to 6 carbon atoms in the straight-chain or branched alkyl part, it being possible for the aryl radical to be monosubstituted to hexasubstituted by identical or different substituents from the series comprising the abovementioned aryl substituents; furthermore represents straight-chain or branched alkyl having 1 to 20 carbon atoms; furthermore represents alkyl having 1 to 20 carbon atoms which is monosubstituted to tetrasubstituted by identical or different substituents from the series comprising halogen, alkoxy having 1 to 6 carbon atoms and alkylthio having 1 to 6 carbon atoms, it being possible for each of the alkyl parts to be straight-chain or branched; in addition represents straight-chain or branched alkenyl having 2 to 16 carbon atoms or alkenyl having 2 to 6 carbon atoms which is monosubstituted to hexasubstituted by identical or different halogen; in addition represents cycloalkyl having 3 to 10 carbon atoms or cycloalkylalkyl having 1 to 10 carbon atoms in the straight-chain or branched alkyl part and 3 to 10 carbon atoms in the cycloalkyl part, it being possible for the cyclic parts, in each case independently, to be monosubstituted to hexasubstituted by identical or different substituents from the series comprising halogen, straight-chain or branched alkyl having 1 to 6 carbon atoms and halogenoalkyl having 1 to 6 carbon atoms and 1 to 9 identical or different halogen atoms, or $R^9$, in addition, represents cycloalkenyl which has 5 to 10 carbon atoms and which may be monosubstituted to hexasubstituted by identical or different, straight-chain or branched alkyl having 1 to 6 carbon atoms;

$R^{10}$ has the meaning of $R^9$ or represents a heterocyclic ring having 5 to 7 ring members, one or

more hetero atoms being included in addition to carbon. The heterocyclic ring may be monosubstituted to hexasubstituted by identical or different, straight-chain or branched alkyl having 1 to 6 carbon atoms;

$Y^1$ to $Y^4$ are identical or different and represent hydrogen, halogen, nitro, cyano, straight-chain or branched alkyl having 1 to 6 carbon atoms, halogenoalkyl having 1 to 6 carbon atoms in the straight-chain or branched alkyl part and 1 to 9 identical or different halogen atoms, alkoxy or halogenoalkoxy, alkylthio, alkylsulphonyl or halogenoalkylthio in each case having 1 to 6 carbon atoms in the straight-chain or branched alkyl part and, if appropriate, 1 to 9 identical or different halogen atoms;

$R^1$ to $R^7$ are identical or different and represent hydrogen, straight-chain or branched alkyl having 1 to 6 carbon atoms, halogenoalkyl having 1 to 6 carbon atoms in the straight-chain or branched alkyl part and 1 to 9 identical or different halogen atoms, or alkoxyalkyl having 1 to 6 carbon atoms in each straight-chain or branched alkyl part;

n represents 0 or 1, and

Z represents straight-chain or branched alkyl or halogenoalkyl each having 1 to 6 carbon atoms and, if appropriate, 1 to 9 identical or different halogen atoms, or the

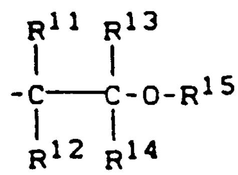

radical, in which

$R^{11}$ to $R^{14}$ are identical or different and represent hydrogen, straight-chain or branched alkyl having 1 to 6 carbon atoms, halogenoalkyl having 1 to 6 carbon atoms in the straight-chain or branched alkyl part and 1 to 9 identical or different halogen atoms, or alkoxyalkyl having 1 to 6 carbon atoms in each straight-chain or branched alkyl part, and

$R^{15}$ represents straight-chain or branched alkyl, alkoxyalkyl or halogenoalkyl each having 1 to 6 carbon atoms per alkyl radical and, if appropriate, 1 to 9 identical or different halogen atoms, and the diastereomers and diastereomer mixtures thereof, characterized in that aminophenols of the formula (II)

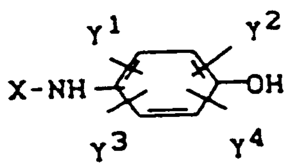

(II)

in which

X and $Y^1$ to $Y^4$ have the abovementioned meanings,

a) are reacted with isocyanates of the formula (III)

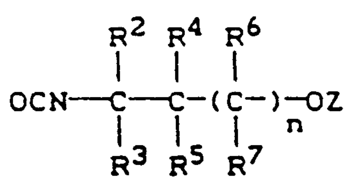

(III)

in which

$R^2$ to $R^7$, n and Z have the abovementioned meanings, if appropriate in the presence of a base and if appropriate in the presence of diluents or solvent, or

b) are reacted with halogenocarbonyl compounds of the formula (IV)

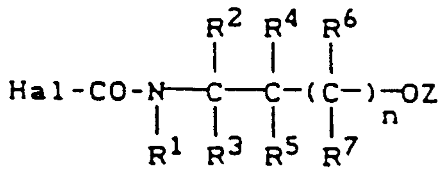

(IV)

in which

$R^1$ to $R^7$, n and Z have the abovementioned meanings, and

Hal represents halogen,

if appropriate in the presence of an acid acceptor and if appropriate in the presence of a solvent or diluent.

**2.** Process according to Claim 1, in which, in the formula (I),

X represents $-CO-NR^8R^9$; $-CO-OR^9$; $-CO-R^{10}$; $-SO_2R^9$ or hydrogen, in which

$R^8$ represents hydrogen,

$R^9$ represents phenyl, benzyl, phenylethyl or 1-phenyl-1-methyl-ethyl each of which is optionally monosubstituted, disubstituted or trisubstituted in the phenyl part by identical or different substituents from the series comprising halogen, straight-chain or branched alkyl having 1 to 4 carbon atoms, straight-chain or branched alkoxy having 1 to 4 carbon atoms, straight-chain or branched alkylthio having 1 to 4 carbon atoms, halogenoalkyl, halogenoalkoxy or halogenoalkylthio in each case having 1 to 4 carbon in the straight-chain or branched alkyl part and 1 to 5 identical or different halogen atoms, cyano or nitro; represents straight-chain or branched alkyl having 1 to 18 carbon atoms; alkyl which has 1 to 4 carbon atoms in the straight-chain or branched alkoxy or alkylthio part and 1 to 10 carbon atoms in the alkyl part and which is monosubstituted, disubstituted or trisubstituted by identical or different substituents from the series comprising halogen, alkoxy or alkylthio; straight-chain or branched alkenyl having 2 to 8 carbon atoms; straight-chain or branched alkenyl having 2 to 4 carbon atoms which is monosubstituted, disubstituted or trisubstituted by identical or different halogen; cycloalkyl which has 3 to 6 carbon atoms or cycloalkyl-alkyl which has 3 to 6 carbon atoms in the cycloalkyl part and 1 to 6 carbon atoms in the straight-chain or branched alkyl part and each of which is optionally monosubstituted, disub-stituted or trisubstituted by identical or different substituents from the series comprising halogen, straight-chain or branched alkyl having 1 to 4 carbon atoms or straight-chain or branched halogenoalkyl having 1 to 4 carbon atoms and 1 to 5 identical or different halogen atoms;

$R^{10}$ has the meaning of $R^9$ or represents a 5- or 6-membered heterocyclic ring which contains one or two oxygen atoms and which is optionally monosubstituted, disubstituted or trisubstituted by straight-chain or branched alkyl having 1 to 4 carbon atoms,

$Y^1$ to $Y^4$ are identical or different and represent hydrogen, halogen, straight-chain or branched alkyl having 1 to 4 carbon atoms, straight-chain or branched alkoxy or alkylsulphonyl each having 1 to 4 carbon atoms, or halogenoalkyl having 1 to 4 carbon atoms in the straight-chain or branched alkyl part and 1 to 5 identical or different halogen atoms;

$R^1$ to $R^7$ are identical or different and represent hydrogen or straight-chain or branched alkyl having 1 to 6 carbon atoms,

n represents 0, and

Z represents straight-chain or branched alkyl or halogenoalkyl each having 1 to 4 carbon atoms and, if appropriate, 1 to 5 identical or different halogen atoms, or the

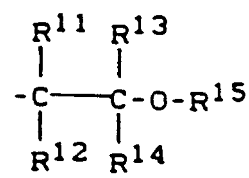

radical, in which

$R^{11}$ to $R^{14}$ are identical or different and represent hydrogen, straight-chain or branched alkyl or halogenoalkyl in each case having 1 to 4 carbon atoms and, if appropriate, 1 to 5 identical or different halogen atoms and

$R^{15}$ represents straight-chain or branched alkyl, alkoxyalkyl or halogenoalkyl in each case having 1

to 4 carbon atoms per alkyl radical and, if appropriate, 1 to 5 identical or different halogen atoms, and the diastereomers and diastereomer mixtures thereof.

**3.** Process according to Claim 1, in which, in the formula (I),

X represents -CO-NHR$^9$; -CO-OR$^9$; -CO-R$^{10}$ or -$SO_2R^9$ in which

$R^9$ represents phenyl which is optionally monosubstituted, disubstituted or trisubstituted by identical or different substituents from the series comprising methyl, ethyl, methoxy, ethoxy, trichloromethyl, trifluoromethyl, trichloromethoxy, trifluoromethoxy, fluorine and chlorine; represents benzyl; straight-chain or branched alkyl having 1 to 5 carbon atoms; halogenoalkyl having 1 to 4 carbon atoms and 1 or 2 chlorine or fluorine atoms; halogenoalkenyl having 2 or 3 carbon atoms and 1 to 4 chlorine atoms; cyclopropyl or cyclohexyl each of which is optionally monosubstituted, disubstituted or trisubstituted by identical or different, straight-chain or branched alkyl having 1 to 4 carbon atoms,

$R^{10}$ represents straight-chain or branched alkyl having 1 to 18 carbon atoms, represents phenyl, cyclopropyl or cyclohexyl each of which is optionally monosubstituted, disubstituted or trisubstituted by identical or different substituents from the series comprising methyl, ethyl, n-propyl, iso-propyl, chlorine or trifluoromethyl; phenylalkyl which has 1 to 3 carbon atoms in the straight-chain or branched alkyl part and which is optionally monosubstituted or disubstituted by chlorine; straight-chain or branched halogenoalkyl having 1 to 4 carbon atoms and 1 or 2 chlorine or fluorine atoms; straight-chain or branched halogenoalkenyl having 2 to 4 carbon atoms and 1 to 4 identical or different halogen atoms, such as chlorine and fluorine, or oxanyl, oxolanyl, dioxanyl or dioxolanyl each of which is optionally monosubstituted by methyl or ethyl;

$Y^1$ to $Y^4$ are identical or different and represent hydrogen, chlorine, fluorine, bromine, methyl, t-butyl, trifluoromethyl or methylsulphonyl,

$R^1$ to $R^7$ are identical or different and represent hydrogen or methyl,

n represents 0, and

Z represents straight-chain or branched alkyl having 1 to 4 carbon atoms, or the

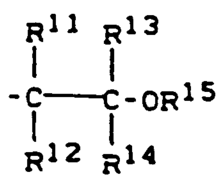

radical, in which

$R^{11}$ to $R^{14}$ represent hydrogen, and

$R^{15}$ represents methyl, ethyl, propyl, butyl, methoxyethyl or ethoxyethyl,

and the diastereomers and diastereomer mixtures thereof.

**4.** Process according to Claims 1 to 3, in which the optionally substituted amino radical is in the 2-, 3- or 4-position to the carbamate radical.

**5.** Pesticides, characterized in that they contain at least one substituted aminophenyl carbamate of the formula (I) according to Claims 1 to 4.

**6.** Use of substituted aminophenyl carbamates of the formula (I) according to Claims 1 to 4 for combating pests.

**7.** Process for combating pests, characterized in that substituted aminophenyl carbamates of the formula (I) according to Claims 1 to 4 are allowed to act on the pests and/or their environment.

**8.** Process for the production of pesticides, characterized in that substituted aminophenyl carbamates of the formula (I) according to Claims 1 to 4 are mixed with extenders and/or surface-active agents.

**9.** Process for the preparation of aminophenols of the formulae (IIA) and (IIB)

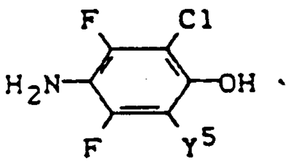 (IIA) and 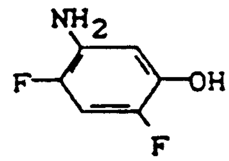 (IIB)

in which

$Y^5$ represents fluorine or chlorine, characterized in that hydroxybenzoic acids of the formulae (VA) and (VB)

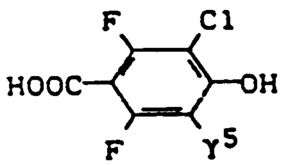 (VA) and 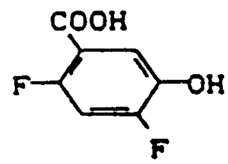 (VB)

are decarboxylated and the resultant phenols of the formulae (VIA) and (VIB)

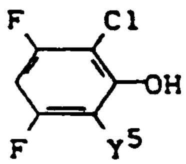 (VIA) and 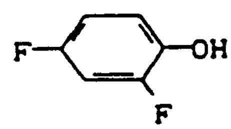 (VIB)

are nitrated to give the compounds of the formulae (VIIA) and (VIIB)

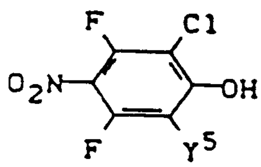 (VIIA) bzw. 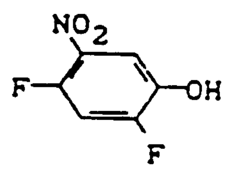 (VIIB)

in which

$Y^5$ represents fluorine or chlorine, and the latter are subsequently hydrogenated.

**Revendications**

**Revendications pour les Etats contractants suivants : BE, CH, DE, FR, GB, IT, LI, NL**

**1.** Carbamates d'aminophényle substitués de formule (I)

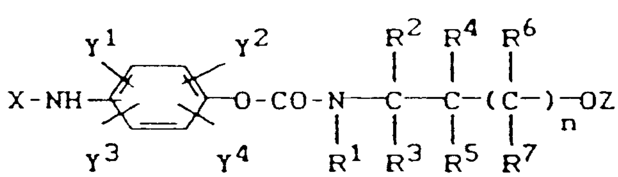 (I)

dans laquelle

X est un groupe $-CO-NR^8R^9$ ; $-CO-OR^9$ ; $-CO-SR^9$ ; $-CO-R^{10}$ ; $-SO_2R^9$ ou l'hydrogène, où

$R^8$ est l'hydrogène ou un groupe alkyle à chaîne droite ou à chaîne ramifiée ayant 1 à 6 atomes de carbone,

$R^9$ est un groupe aryle ayant 6 à 12 atomes de carbone, un groupe aryle de 1 à 6 atomes de carbone portant 1 à 6 substituants, identiques ou différents, halogéno, alkyle ayant 1 à 6 atomes de carbone, alkoxy ayant 1 à 6 atomes de carbone, alkylthio ayant 1 à 6

atomes de carbone, halogénalkyle ayant 1 à 6 atomes de carbone et 1 à 9 atomes d'halogènes identiques ou différents, halogénalkoxy ayant 1 à 6 atomes de carbone et 1 à 9 atomes d'halogènes identiques ou différents, halogénalkylthio ayant 1 à 6 atomes de carbone et 1 à 9 atomes d'halogènes identiques ou différents, cyano et nitro ; en outre un groupe aralkyle de 6 à 12 atomes de carbone dans la partie aryle et de 1 à 6 atomes de carbone dans la partie alkyle à chaîne droite ou ramifiée, le reste aryle pouvant porter 1 à 6 des substituants aryle mentionnés ci-dessus, identiques ou différents, en outre un groupe alkyle à chaîne droite ou ramifiée ayant 1 à 20 atomes de carbone ; un groupe alkyle de 1 à 20 atomes de carbone portant 1 à 4 substituants, identiques ou différents, halogéno, alkoxy ayant 1 à 6 atomes de carbone ou alkylthio ayant 1 à 6 atomes de carbone, les parties alkyle pouvant chacune être à chaîne droite ou ramifiée ;

en outre un groupe alcényle à chaîne droite ou ramifiée ayant 2 à 16 atomes de carbone ou un groupe alcényle de 2 à 6 atomes de carbone portant 1 à 6 substituants halogéno identiques ou différents ; en outre un groupe cycloalkyle ayant 3 à 10 atomes de carbone ou un groupe cycloalkyl-alkyle ayant 1 à 10 atomes de carbone dans la partie alkyle à chaîne droite ou ramifiée et 3 à 10 atomes de carbone dans la partie cycloalkyle, les parties cycliques pouvant porter chacune indépendamment 1 à 6 substituants, identiques ou différents, halogéno, alkyle à chaîne droite ou ramifiée ayant 1 à 6 atomes de carbone et halogénalkyle ayant 1 à 6 atomes de carbone et 1 à 9 atomes d'halogènes identiques ou différents, ou bien $R^9$ représente en outre un groupe cycloalcényle de 5 à 10 atomes de carbone, qui peut porter 1 à 6 substituants, identiques ou différents, alkyle à chaîne droite ou ramifiée ayant 1 à 6 atomes de carbone ;

$R^{10}$ a la définition de $R^9$ ou représente un hétérocycle de 5 à 7 chaînons, contenant en plus de carbone un ou plusieurs hétéroatomes, l'hétérocycle pouvant porter 1 à 6 substituants, identiques ou différents, alkyle à chaîne droite ou ramifiée ayant 1 à 6 atomes de carbone ;

$Y^1$ à $Y^4$ sont identiques ou différents et représentent l'hydrogène, un halogène, un groupe nitro, cyano, alkyle à chaîne droite ou ramifiée ayant 1 à 6 atomes de carbone, halogénalkyle ayant 1 à 6 atomes de carbone dans la partie alkyle à chaîne droite ou ramifiée et 1 à 9 atomes d'halogènes identiques ou différents, alkoxy ou halogénalkoxy, alkylthio, alkylsulfonyle ou halogénalkylthio ayant chacun 1 à 6 atomes de carbone dans la partie alkyle à chaîne droite ou ramifiée et le cas échéant 1 à 9 atomes d'halogènes identiques ou différents ;

$R^1$ à $R^7$ sont identiques ou différents et représentent l'hydrogène, un groupe alkyle à chaîne droite ou ramifiée ayant 1 à 6 atomes de carbone, halogénalkyle ayant 1 à 6 atomes de carbone dans la partie alkyle à chaîne droite ou ramifiée et 1 à 9 atomes d'halogènes identiques ou différents ou un groupe alkoxyalkyle ayant 1 à 6 atomes de carbone dans chaque partie alkyle à chaîne droite ou ramifiée ;

n a la valeur 0 ou 1 et

Z est un groupe alkyle ou halogénalkyle à chaîne droite ou ramifiée ayant chacun 1 à 6 atomes de carbone et le cas échéant 1 à 9 atomes d'halogènes identiques ou différents ou le reste

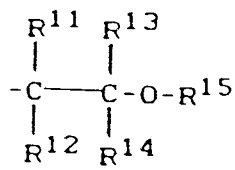

,

dans lequel

$R^{11}$ à $R^{14}$ sont identiques ou différents et représentent de l'hydrogène, un groupe alkyle à chaîne droite ou ramifiée ayant 1 à 6 atomes de carbone, un groupe halogénalkyle ayant 1 à 6 atomes de carbone dans la partie alkyle à chaîne droite ou ramifiée et 1 à 9 atomes d'halogènes identiques ou différents ou un groupe alkoxyalkyle ayant 1 à 6 atomes de

carbone par partie alkyle à chaîne droite ou ramifiée et

$R^{15}$ est un groupe alkyle, alkoxyalkyle ou halogénalkyle à chaîne droite ou ramifiée ayant 1 à 6 atomes de carbone par reste alkyle et le cas échéant 1 à 9 atomes d'halogènes identiques ou différents,

leurs diastéréoisomères et des mélanges de leurs diastéréoisomères.

**2.** Carbamates d'aminophényle substitués suivant la revendication 1, dans la formule (I) de laquelle

X est un groupe $-CO-NR^8R^9$ ; $-CO-OR^9$ ; $-CO-R^{10}$ ; $-SO_2R^9$ ou l'hydrogène, où

$R^8$ représente l'hydrogène,

$R^9$ est un groupe phényle, benzyle, phényléthyle ou 1-phényl-1-méthyléthyle portant chacun le cas échéant dans la partie phényle 1 à 3 substituants, identiques ou différents, halogéno, alkyle à chaîne droite ou ramifiée ayant 1 à 4 atomes de carbone, alkoxy à chaîne droite ou ramifiée ayant 1 à 4 atomes de carbone, alkylthio à chaîne droite ou ramifiée ayant 1 à 4 atomes de carbone, halogénalkyle, halogénalkoxy ou halogénalkylthio ayant chacun 1 à 4 atomes de carbone dans la partie alkyle à chaîne droite ou ramifiée et 1 à 5 atomes d'halogènes identiques ou différents, cyano ou nitro ;

un groupe alkyle à chaîne droite ou ramifiée ayant 1 à 18 atomes de carbone ; un groupe alkyle portant 1 à 3 substituants, identiques ou différents, halogéno, alkoxy ou alkylthio ayant 1 à 4 atomes de carbone dans la partie alkoxy ou alkylthio à chaîne droite ou ramifiée et 1 à 10 atomes de carbone dans la partie alkyle ; un groupe alcényle à chaîne droite ou ramifiée ayant 2 à 8 atomes de carbone ; un groupe alcényle de 2 à 4 atomes de carbone portant 1 à 3 substituants halogéno identiques ou différents, un groupe cycloalkyle de 3 à 6 atomes de carbone ou cycloalkyl-alkyle de 3 à 6 atomes de carbone dans la partie cycloalkyle et 1 à 6 atomes de carbone dans la partie alkyle à chaîne droite ou ramifiée, portant éventuellement 1 à 3 substituants, identiques ou différents, halogéno, alkyle à chaîne droite ou ramifiée ayant 1 à 4 atomes de carbone ou halogénalkyle à chaîne droite ou ramifiée ayant 1 à 4 atomes de carbone et 1 à 5 atomes d'halogènes identiques ou différents ;

$R^{10}$ a la définition de $R^9$ ou représente un hétérocycle pentagonal ou hexagonal comprenant 1 ou 2 atomes d'oxygène, portant éventuellement 1 à 3 substituants alkyle à chaîne droite ou ramifiée ayant 1 à 4 atomes de carbone,

$Y^1$ à $Y^4$ sont identiques ou différents et représentent l'hydrogène, un halogène, un groupe alkyle à chaîne droite ou ramifiée ayant 1 à 4 atomes de carbone, un groupe alkoxy ou alkylsulfonyle à chaîne droite ou ramifiée ayant chacun 1 à 4 atomes de carbone ou un groupe halogénalkyle ayant 1 à 4 atomes de carbone dans la partie alkyle à chaîne droite ou ramifiée et 1 à 5 atomes d'halogènes identiques ou différents ;

$R^1$ à $R^7$ sont identiques ou différents et représentent l'hydrogène ou un groupe alkyle à chaîne droite ou ramifiée ayant 1 à 6 atomes de carbone,

n a la valeur 0 et

Z est un groupe alkyle ou halogénalkyle à chaîne droite ou ramifiée ayant chacun 1 à 4 atomes de carbone et le cas échéant 1 à 5 atomes d'halogènes identiques ou différents ou le reste

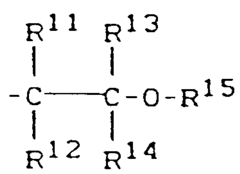

,

dans lequel

$R^{11}$ à $R^{14}$ sont identiques ou différents et représentent l'hydrogène, un groupe alkyle ou halogénalkyle à chaîne droite ou ramifiée ayant chacun 1 à 4 atomes de carbone et le cas échéant 1 à 5 atomes d'halogènes identiques ou différents et

$R^{15}$ est un groupe alkyle, alkoxyalkyle ou halogénalkyle à chaîne droite ou ramifiée ayant chacun 1 à 4 atomes de carbone par reste alkyle et le cas échéant 1 à 5 atomes

d'halogènes identiques ou différents,
leurs diastéréoisomères et des mélanges de leurs diastéréoisomères.

**3.** Carbamates d'aminophényle substitués suivant la revendication 1, dans la formule (I) de laquelle

X est un groupe -CO-NHR$^9$ ; CO-OR$^9$ ; -CO-R$^{10}$ ou -$SO_2R^9$, où

$R^9$ est un groupe phényle portant éventuellement 1 à 3 substituants, identiques ou différents, méthyle, éthyle, méthoxy, éthoxy, trichlorométhyle, trifluorométhyle, trichlorométhoxy, trifluorométhoxy, fluoro et chloro ; un groupe benzyle ; un groupe alkyle à chaîne droite ou ramifiée ayant 1 à 5 atomes de carbone ; un groupe halogénalkyle ayant 1 à 4 atomes de carbone et 1 ou 2 atomes de chlore ou de fluor ; un groupe halogénalkyle ayant 2 ou 3 atomes de carbone et 1 à 4 atomes de chlore ; un groupe cyclopropyle ou cyclohexyle portant chacun le cas échéant 1 à 3 substituants, identiques ou différents, alkyle à chaîne droite ou ramifiée ayant 1 à 4 atomes de carbone,

$R^{10}$ est un groupe alkyle à chaîne droite ou ramifiée ayant 1 à 18 atomes de carbone, un groupe phényle, cyclopropyle ou cyclohexyle portant chacun le cas échéant 1 à 3 substituants méthyle, éthyle, n-propyle, isopropyle, chloro ou trifluorométhyle identiques ou différents ; un groupe phénylalkyle ayant 1 à 3 atomes de carbone dans la partie alkyle à chaîne droite ou ramifiée portant éventuellement 1 ou 2 substituants chloro, un groupe halogénalkyle à chaîne droite ou ramifiée ayant 1 à 4 atomes de carbone et 1 ou 2 atomes de chlore ou de fluor ; un groupe halogénalcényle à chaîne droite ou ramifiée ayant 2 à 4 atomes de carbone et 1 à 4 atomes d'halogènes identiques ou différents tels que chlore et fluor ou un groupe oxannyle, oxolanyle, dioxannyle ou dioxolanyle portant éventuellement un substituant méthyle ou éthyle ;

$Y^1$ à $Y^4$ sont identiques ou différents et représentent l'hydrogène, le chlore, le fluor, le brome, un groupe méthyle, tertiobutyle, trifluorométhyle ou méthylsulfonyle,

$R^1$ à $R^7$ sont identiques ou différents et représentent l'hydrogène ou un groupe méthyle,

n a la valeur 0 et

Z représente un groupe alkyle à chaîne droite ou ramifiée ayant 1 à 4 atomes de carbone ou le reste

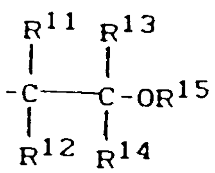,

dans lequel

$R^{11}$ à $R^{14}$ représentent l'hydrogène et

$R^{15}$ est un groupe méthyle, éthyle, propyle, butyle, méthoxyéthyle ou éthoxyéthyle,

leurs diastéréoisomères et des mélanges de leurs diastéréoisomères.

**4.** Procédé de production de carbamates d'aminophényle substitués de formule (I)

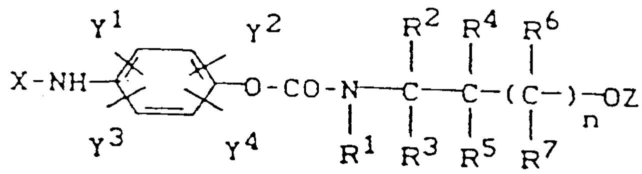

(I)

dans laquelle
n, X, $Y^1$-$Y^4$, Z et $R^1$-$R^7$ ont la définition indiquée dans la revendication 1,
caractérisé en ce qu'on fait réagir des aminophénols de formule (II)

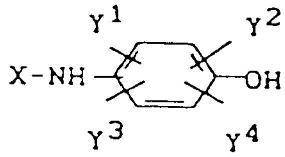

(II)

dans laquelle

X et $Y^1$ à $Y^4$ ont les définitions indiquées ci-dessus,

a) avec des isocyanates de formule (III)

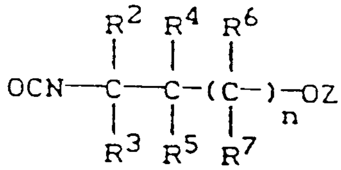

(III)

dans laquelle

$R^2$ à $R^7$, n et Z ont les définitions indiquées ci-dessus, le cas échéant en présence d'une base et en la présence éventuelle de diluants ou de solvants, ou bien

b) avec des composés halogénocarbonyliques de formule (IV)

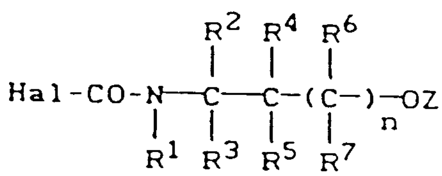

(IV)

dans laquelle

$R^1$ à $R^7$, n et Z ont les définitions indiquées ci-dessus et

Hal représente un halogène,

le cas échéant en présence d'un accepteur d'acide et en la présence éventuelle d'un solvant ou d'un diluant.

**5.** Composés de formule (I) suivant les revendications 1 à 4, dans lesquels le reste amino éventuellement substitué est en position 2, 3 ou 4 par rapport au reste carbamate.

**6.** Compositions pesticides, caractérisées par une teneur en au moins un carbamate d'aminophényle substitué de formule (I) suivant les revendications 1 ou 4.

**7.** Utilisation de carbamates d'aminophényle substitués de formule (I) suivant les revendications 1 ou 4 pour combattre des parasites.

**8.** Procédé pour combattre des parasites, caractérisé en ce qu'on fait agir des carbamates d'aminophényle substitués de formule (I) suivant les revendications 1 ou 4 sur les parasites et/ou sur leur milieu.

**9.** Procédé de préparation de compositions pesticides, caractérisé en ce qu'on mélange des carbamates d'aminophényle substitués de formule (I) suivant les revendications 1 ou 4 avec des diluants et/ou des agents tensioactifs.

**10.** Aminophénols de formule (IIA) ou (IIB)

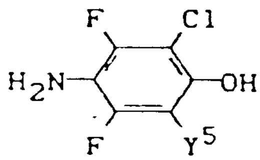 (IIA) ou 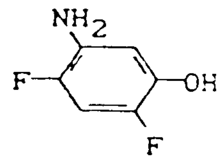 (IIB)

dans laquelle

$Y^5$ représente le fluor ou le chlore.

**11.** Nitrophénols de formules (VIIA) ou (VIIB)

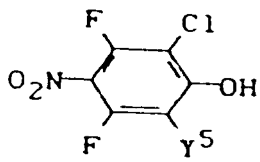 (VIIA) ou 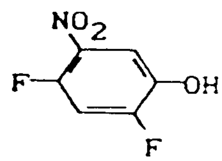 (VIIB)

dans lesquelles

$Y^5$ représente le fluor ou le chlore.

**12.** Procédé de production d'aminophénols de formules (IIA) et (IIB)

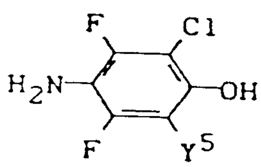 (IIA) et 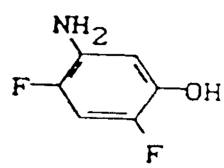 (IIB)

dans lesquelles

$Y^5$ représente le fluor ou le chlore,

caractérisé en ce qu'on décarboxyle des acides hydroxybenzoïques de formules (VA) et respectivement (VB)

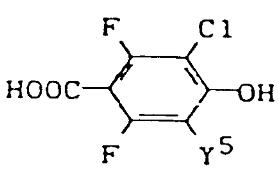 (VA) et 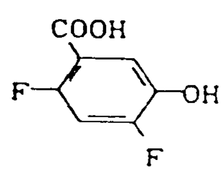 (VB)

et on nitre les phénols produits de formules (VIA) et (VIB)

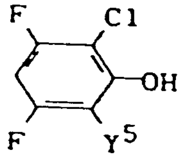 (VIA) et 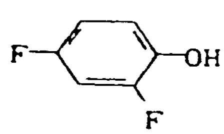 (VIB)

en les composés de formules (VIIA) et (VIIB)

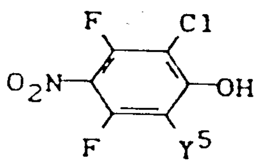 (VIIA) et 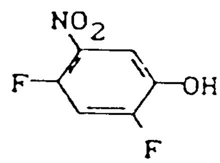 (VIIB)

dans lesquelles

$Y^5$ représente le fluor ou le chlore,

puis on les hydrogène.

**Revendications pour l'Etat contractant suivant : ES**

**1.** Procédé de production de carbamates d'aminophényle substitués de formule (I)

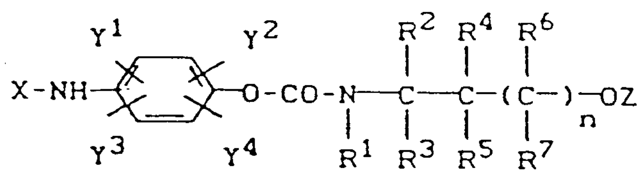 (I)

dans laquelle

X est un groupe $-CO-NR^8R^9$ ; $-CO-OR^9$ ; $-CO-SR^9$ ; $-CO-R^{10}$ ; $-SO_2R^9$ ou l'hydrogène, où

$R^8$ est l'hydrogène ou un groupe alkyle à chaîne droite ou à chaîne ramifiée ayant 1 à 6 atomes de carbone,

$R^9$ est un groupe aryle ayant 6 à 12 atomes de carbone, un groupe aryle de 1 à 6 atomes de carbone portant 1 à 6 substituants, identiques ou différents, halogéno, alkyle ayant 1 à 6 atomes de carbone, alkoxy ayant 1 à 6 atomes de carbone, alkylthio ayant 1 à 6 atomes de carbone, halogénalkyle ayant 1 à 6 atomes de carbone et 1 à 9 atomes d'halogènes identiques ou différents, halogénalkoxy ayant 1 à 6 atomes de carbone et 1 à 9 atomes d'halogènes identiques ou différents, halogénalkylthio ayant 1 à 6 atomes de carbone et 1 à 9 atomes d'halogènes identiques ou différents, cyano et nitro ; en outre un groupe aralkyle de 6 à 12 atomes de carbone dans la partie aryle et de 1 à 6 atomes de carbone dans la partie alkyle à chaîne droite ou ramifiée, le reste aryle pouvant porter 1 à 6 des substituants aryle mentionnés ci-dessus, identiques ou différents, en outre un groupe alkyle à chaîne droite ou ramifiée ayant 1 à 20 atomes de carbone ; un groupe alkyle de 1 à 20 atomes de carbone portant 1 à 4 substituants, identiques ou différents, halogéno, alkoxy ayant 1 à 6 atomes de carbone ou alkylthio ayant 1 à 6 atomes de carbone, les parties alkyle pouvant chacune être à chaîne droite ou ramifiée ;

en outre un groupe alcényle à chaîne droite ou ramifiée ayant 2 à 16 atomes de carbone ou un groupe alcényle de 2 à 6 atomes de carbone portant 1 à 6 substituants halogéno identiques ou différents ; en outre un groupe cycloalkyle ayant 3 à 10 atomes de carbone ou un groupe cycloalkyl-alkyle ayant 1 à 10 atomes de carbone dans la partie alkyle à chaîne droite ou ramifiée et 3 à 10 atomes de carbone dans la partie cycloalkyle, les parties cycliques pouvant porter chacune indépendamment 1 à 6 substituants, identiques ou différents, halogéno, alkyle à chaîne droite ou ramifiée ayant 1 à 6 atomes de carbone et halogénalkyle ayant 1 à 6 atomes de carbone et 1 à 9 atomes d'halogènes identiques ou différents, ou bien $R^9$ représente en outre un groupe cycloalcényle de 5 à 10 atomes de carbone, qui peut porter 1 à 6 substituants, identiques ou différents, alkyle à chaîne droite ou ramifiée ayant 1 à 6 atomes de carbone ;

$R^{10}$ a la définition de $R^9$ ou représente un hétérocycle de 5 à 7 chaînons, contenant en

plus de carbone un ou plusieurs hétéroatomes, l'hétérocycle pouvant porter 1 à 6 substituants, identiques ou différents, alkyle à chaîne droite ou ramifiée ayant 1 à 6 atomes de carbone ;

$Y^1$ à $Y^4$ sont identiques ou différents et représentent l'hydrogène, un halogène, un groupe nitro, cyano, alkyle à chaîne droite ou ramifiée ayant 1 à 6 atomes de carbone, halogénalkyle ayant 1 à 6 atomes de carbone dans la partie alkyle à chaîne droite ou ramifiée et 1 à 9 atomes d'halogènes identiques ou différents, alkoxy ou halogénalkoxy, alkylthio, alkylsulfonyle ou halogénalkylthio ayant chacun 1 à 6 atomes de carbone dans la partie alkyle à chaîne droite ou ramifiée et le cas échéant 1 à 9 atomes d'halogènes identiques ou différents ;

$R^1$ à $R^7$ sont identiques ou différents et représentent l'hydrogène, un groupe alkyle à chaîne droite ou ramifiée ayant 1 à 6 atomes de carbone, halogénalkyle ayant 1 à 6 atomes de carbone dans la partie alkyle à chaîne droite ou ramifiée et 1 à 9 atomes d'halogènes identiques ou différents ou un groupe alkoxyalkyle ayant 1 à 6 atomes de carbone dans chaque partie alkyle à chaîne droite ou ramifiée ;

n a la valeur 0 ou 1 et

Z est un groupe alkyle ou halogénalkyle à chaîne droite ou ramifiée ayant chacun 1 à 6 atomes de carbone et le cas échéant 1 à 9 atomes d'halogènes identiques ou différents ou le reste

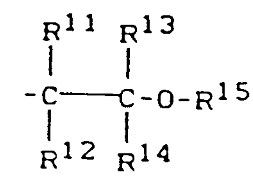 ,

dans lequel

$R^{11}$ à $R^{14}$ sont identiques ou différents et représentent de l'hydrogène, un groupe alkyle à chaîne droite ou ramifiée ayant 1 à 6 atomes de carbone, un groupe halogénalkyle ayant 1 à 6 atomes de carbone dans la partie alkyle à chaîne droite ou ramifiée et 1 à 9 atomes d'halogènes identiques ou différents ou un groupe alkoxyalkyle ayant 1 à 6 atomes de carbone par partie alkyle à chaîne droite ou ramifiée et

$R^{15}$ est un groupe alkyle, alkoxyalkyle ou halogénalkyle à chaîne droite ou ramifiée ayant 1 à 6 atomes de carbone par reste alkyle et le cas échéant 1 à 9 atomes d'halogènes identiques ou différents,

et de leurs diastéréoisomères et de leurs mélanges de diastéréoisomères, caractérisé en ce qu'on fait réagir des aminophénols de formule (II)

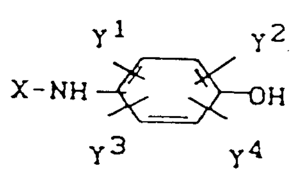 (II)

dans laquelle

X et $Y^1$ à $Y^4$ ont les définitions indiquées ci-dessus,

a) avec des isocyanates de formule (III)

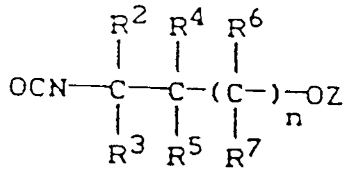

(III)

dans laquelle
$R^2$ à $R^7$, n et Z ont les définitions indiquées ci-dessus, éventuellement en présence d'une base et en la présence éventuelle de diluants ou de solvants, ou bien
b) avec des composés halogénocarbonyliques de formule (IV)

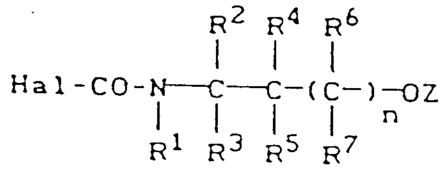

(IV)

dans laquelle
$R^1$ à $R^7$, n et Z ont les définitions indiquées ci-dessus et
Hal est un halogène,
éventuellement en présence d'un accepteur d'acide et en la présence éventuelle d'un solvant ou d'un diluant.

**2.** Procédé suivant la revendication 1, dans lequel, dans la formule (I)

X est un groupe $-CO-NR^8R^9$ ; $-CO-OR^9$ ; $-CO-R^{10}$ ; $-SO_2R^9$ ou l'hydrogène, où

$R^8$ représente l'hydrogène,

$R^9$ est un groupe phényle, benzyle, phényléthyle ou 1-phényl-1-méthyléthyle portant chacun le cas échéant dans la partie phényle 1 à 3 substituants, identiques ou différents, halogéno, alkyle à chaîne droite ou ramifiée ayant 1 à 4 atomes de carbone, alkoxy à chaîne droite ou ramifiée ayant 1 à 4 atomes de carbone, alkylthio à chaîne droite ou ramifiée ayant 1 à 4 atomes de carbone, halogénalkyle, halogénalkoxy ou halogénalkylthio ayant chacun 1 à 4 atomes de carbone dans la partie alkyle à chaîne droite ou ramifiée et 1 à 5 atomes d'halogènes identiques ou différents, cyano ou nitro ;
un groupe alkyle à chaîne droite ou ramifiée ayant 1 à 18 atomes de carbone ; un groupe alkyle portant 1 à 3 substituants, identiques ou différents, halogéno, alkoxy ou alkylthio ayant 1 à 4 atomes de carbone dans la partie alkoxy ou alkylthio à chaîne droite ou ramifiée et 1 à 10 atomes de carbone dans la partie alkyle ; un groupe alcényle à chaîne droite ou ramifiée ayant 2 à 8 atomes de carbone ; un groupe alcényle de 2 à 4 atomes de carbone portant 1 à 3 substituants halogéno identiques ou différents, un groupe cycloalkyle de 3 à 6 atomes de carbone ou cycloalkyl-alkyle de 3 à 6 atomes de carbone dans la partie cycloalkyle et 1 à 6 atomes de carbone dans la partie alkyle à chaîne droite ou ramifiée, portant éventuellement 1 à 3 substituants, identiques ou différents, halogéno, alkyle à chaîne droite ou ramifiée ayant 1 à 4 atomes de carbone ou halogénalkyle à chaîne droite ou ramifiée ayant 1 à 4 atomes de carbone et 1 à 5 atomes d'halogènes identiques ou différents ;

$R^{10}$ a la définition de $R^9$ ou représente un hétérocycle pentagonal ou hexagonal comprenant 1 ou 2 atomes d'oxygène, portant éventuellement 1 à 3 substituants alkyle à chaîne droite ou ramifiée ayant 1 à 4 atomes de carbone,

$Y^1$ à $Y^4$ sont identiques ou différents et représentent l'hydrogène, un halogène, un groupe alkyle à chaîne droite ou ramifiée ayant 1 à 4 atomes de carbone, un groupe alkoxy ou alkylsulfonyle à chaîne droite ou ramifiée ayant chacun 1 à 4 atomes de carbone ou un groupe halogénalkyle ayant 1 à 4 atomes de carbone dans la partie alkyle à chaîne

droite ou ramifiée et 1 à 5 atomes d'halogènes identiques ou différents ;

$R^1$ à $R^7$ sont identiques ou différents et représentent l'hydrogène ou un groupe alkyle à chaîne droite ou ramifiée ayant 1 à 6 atomes de carbone,

n a la valeur 0 et

Z est un groupe alkyle ou halogénalkyle à chaîne droite ou ramifiée ayant chacun 1 à 4 atomes de carbone et le cas échéant 1 à 5 atomes d'halogènes identiques ou différents ou le reste

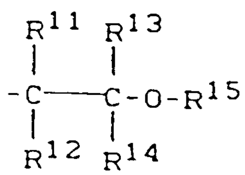

,

dans lequel

$R^{11}$ à $R^{14}$ sont identiques ou différents et représentent l'hydrogène, un groupe alkyle ou halogénalkyle à chaîne droite ou ramifiée ayant chacun 1 à 4 atomes de carbone et le cas échéant 1 à 5 atomes d'halogènes identiques ou différents et

$R^{15}$ est un groupe alkyle, alkoxyalkyle ou halogénalkyle à chaîne droite ou ramifiée ayant chacun 1 à 4 atomes de carbone par reste alkyle et le cas échéant 1 à 5 atomes d'halogènes identiques ou différents,

leurs diastéréoisomères et des mélanges de leurs diastéréoisomères.

**3.** Procédé suivant la revendication 1, dans lequel, dans la formule (I)

X est un groupe $-CO-NHR^9$ ; $-CO-OR^9$ ; $-CO-R^{10}$ ou $-SO_2R^9$, où

$R^9$ est un groupe phényle portant éventuellement 1 à 3 substituants, identiques ou différents, méthyle, éthyle, méthoxy, éthoxy, trichlorométhyle, trifluorométhyle, trichlorométhoxy, trifluorométhoxy, fluoro et chloro ; un groupe benzyle ; un groupe alkyle à chaîne droite ou ramifiée ayant 1 à 5 atomes de carbone ; un groupe halogénalkyle ayant 1 à 4 atomes de carbone et 1 ou 2 atomes de chlore ou de fluor ; un groupe halogénalkyle ayant 2 ou 3 atomes de carbone et 1 à 4 atomes de chlore ; un groupe cyclopropyle ou cyclohexyle portant chacun le cas échéant 1 à 3 substituants, identiques ou différents, alkyle à chaîne droite ou ramifiée ayant 1 à 4 atomes de carbone,

$R^{10}$ est un groupe alkyle à chaîne droite ou ramifiée ayant 1 à 18 atomes de carbone, un groupe phényle, cyclopropyle ou cyclohexyle portant chacun le cas échéant 1 à 3 substituants méthyle, éthyle, n-propyle, isopropyle, chloro ou trifluorométhyle identiques ou différents ; un groupe phénylalkyle ayant 1 à 3 atomes de carbone dans la partie alkyle à chaîne droite ou ramifiée portant éventuellement 1 ou 2 substituants chloro, un groupe halogénalkyle à chaîne droite ou ramifiée ayant 1 à 4 atomes de carbone et 1 ou 2 atomes de chlore ou de fluor ; un groupe halogénalcényle à chaîne droite ou ramifiée ayant 2 à 4 atomes de carbone et 1 à 4 atomes d'halogènes identiques ou différents tels que chlore et fluor ou un groupe oxannyle, oxolanyle, dioxannyle ou dioxolanyle portant éventuellement un substituant méthyle ou éthyle ;

$Y^1$ à $Y^4$ sont identiques ou différents et représentent l'hydrogène, le chlore, le fluor, le brome, un groupe méthyle, tertiobutyle, trifluorométhyle ou méthylsulfonyle,

$R^1$ à $R^7$ sont identiques ou différents et représentent l'hydrogène ou un groupe méthyle,

n a la valeur 0 et

Z représente un groupe alkyle à chaîne droite ou ramifiée ayant 1 à 4 atomes de carbone ou le reste

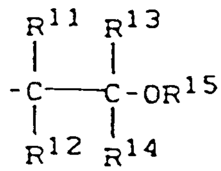
,

dans lequel

$R^{11}$ à $R^{14}$ représentent l'hydrogène et

$R^{15}$ est un groupe méthyle, éthyle, propyle, butyle, méthoxyéthyle ou éthoxyéthyle,

leurs diastéréoisomères et des mélanges de leurs diastéréoisomères.

**4.** Procédé suivant les revendications 1 à 3, dans lequel le reste amino éventuellement substitué se trouve en positions 2, 3 ou 4 par rapport au reste carbamate.

**5.** Compositions pesticides, caractérisées par une teneur en au moins un carbamate aminophénylique substitué de formule (I) suivant les revendications 1 à 4.

**6.** Utilisation de carbamates d'aminophényle substitués de formule (I) suivant les revendications 1 à 4 pour combattre des parasites.

**7.** Procédé pour combattre des parasites, caractérisé en ce qu'on fait agir des carbamates d'aminophényle substitués de formule (I) suivant les revendications 1 à 4 sur les parasites et/ou sur leur milieu.

**8.** Procédé de préparation de compositions pesticides, caractérisé en ce qu'on mélange des carbamates d'aminophényle substitués de formule (I) suivant les revendications 1 à 4 avec des diluants et/ou des agents tensioactifs.

**9.** Procédé de production d'aminophénols de formules (IIA) et (IIB)

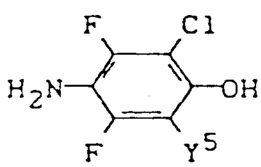 (IIA) et 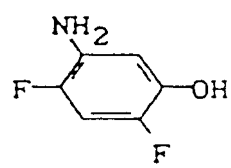 (IIB)

dans lesquelles

$Y^5$ représente le fluor ou le chlore,

caractérisé en ce qu'on décarboxyle des acides hydroxybenzoïques de formules (VA) et (VB)

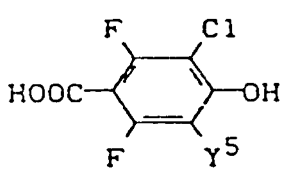 (VA) et 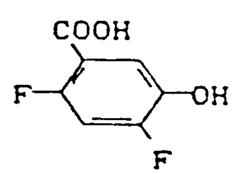 (VB)

et on nitre les phénols produits de formules (VIA) et (VIB)

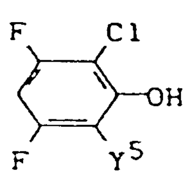 (VIA) et 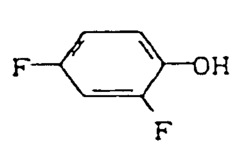 (VIB)

en les composés de formules (VIIA) et (VIIB)

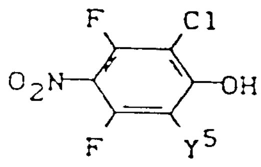 (VIIA) et 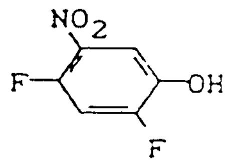 (VIIB)

dans lesquelles

$Y^5$ représente le fluor ou le chlore,

puis on hydrogène ces composés.